# EUROPEAN PATENT APPLICATION

(11) **EP 3 739 338 A2**
(43) Date of publication of application: **18.11.2020**
(21) Application number: 20174852.2
(22) Date of filing: 15.05.2020
(51) Int. Cl.: G01N 33/574

(54) **METHODS FOR PREDICTING IMMUNOTHERAPY RESPONSE OF SUBJECT HAVING CANCER**

(30) Priority: 15.05.2019 US 201962847960 P
(71) Applicant: MiCareo Taiwan Co., Ltd., 114 Taipei City (TW)
(72) Inventor: LEE, Chia-Ying, 114 Taipei City (TW); TSENG, Ju-Yu, 114 Taipei City (TW); WANG, Hong-Ling, 114 Taipei City (TW); WANG, Shin-Hang, 114 Taipei City (TW); CHEN, Jui-Lin, 114 Taipei City (TW)
(74) Representative: Becker, Eberhard

(57) **Abstract**

A method for predicting immunotherapy response of a subject having cancer includes the following steps. A peripheral blood sample is obtained from the subject having cancer before or after receiving the immunotherapy. The number of immune cells in the peripheral blood sample of the subject having cancer is detected. The number of immune cells and a first cut-off value/or a second cut-off value are compared to indicate whether the subject having cancer benefits from the immunotherapy. The first cut-off value/or the second cut-off value is determined by the following steps: a statistical analysis of a correlation between the number of immune cells in a group of subjects having cancer and an expected risk of disease progression in the group of subjects having cancer is performed, and then a statistically significant value used to define the correlation is obtained.

## Description

### BACKGROUND

### Technical Field

The invention relates to a method for predicting cancer therapy response of a subject having cancer, and more particularly, to a method for predicting anti-PD-1/PD-L1 immunotherapy response of a subject having cancer.

### Description of Related Art

Currently, immunotherapy using immune checkpoint blockade (such as PD-1 / PD-L1 inhibitors, but is not limited thereto) may activate the immune system of cancer patients, and then kill tumor cells through the immune response. Therefore, compared with chemotherapy, the cancer patients who respond to immune checkpoint inhibitors may have a longer survival time and a better quality of life. However, immunotherapy using immune checkpoint inhibitors still has the following disadvantages: the treatment is expensive, the response after treatment is slow, and the immune checkpoint blockade is not effective for all cancer patients. At present, there is still no method for predicting treatment effectiveness of the immune checkpoint blockade on cancer patients before administration or evaluating treatment effectiveness of the immune checkpoint blockade on cancer patients after administration.

### SUMMARY

The present invention provides a method for predicting immunotherapy response of a subject having cancer, which may predict treatment effectiveness before the first treatment, and may effectively help the subject having cancer decide whether receiving immunotherapy or not.

The present invention provides another method for predicting immunotherapy response of a subject having cancer, which may determine treatment effectiveness after the previous treatment and before the next treatment, and may effectively help the subject having cancer decide whether continually receiving immunotherapy or not.

The method for predicting the immunotherapy response of the subject having cancer of the invention includes the following steps. A peripheral blood sample is obtained from the subject having cancer before receiving the immunotherapy. The number of immune cells in the peripheral blood sample of the subject having cancer is detected. The number of immune cells is compared with a first cut-off value to indicate whether the subject having cancer benefits from the immunotherapy. The first cut-off value is determined by the following steps: a statistical analysis of a correlation between the number of immune cells in a group of subjects having cancer and an expected risk of disease progression in the group of subjects having cancer is performed, and then a statistically significant value used to define the correlation is obtained.

In an embodiment of the invention, the immunotherapy includes immune checkpoint blockade or cellular immunotherapy.

In an embodiment of the invention, the immune cells express at least one marker of: PD1, CD8, CD4, IFN-γ, TIM3, LAG3, CD25, TGF-β.

In an embodiment of the invention, the cancer is hepatocellular carcinoma and the immune cells are selected from the group consisting of PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CDS⁺IFNγ⁺LAG3⁺ cells and PD1⁺CD8⁺IFNγ⁺TIM3⁺LAG3⁻ cells.

In an embodiment of the invention, the cancer is renal cell carcinoma and the immune cells are selected from the group consisting of PD1⁺CD4⁺TGFβ⁺CD25⁺ cells, PD1⁺CD4⁺TGFβ⁺CD25⁺LAG3⁻ cells and PD1⁺CD4⁺TGFβ⁺CD25⁺LAG3⁺ cells.

In an embodiment of the invention, the first cut-off value divides the subject into group A and group B according to a pattern of hazard ratio, wherein the subject classified into group A has a good prognosis, and the subject classified into group B has worse prognosis.

In an embodiment of the invention, the hazard ratio is measured by a Cox regression model of a survival time in the group of subjects having cancer versus a survival probability in the group of subjects having cancer.

Another method for predicting the immunotherapy response of the subject having cancer of the invention includes the following steps. A peripheral blood sample is obtained from the subject receiving the immunotherapy between the end of one round of treatment until the start of the next round of treatment. The number of immune cells in the peripheral blood sample of the subject having cancer is detected. The number of immune cells is compared with a second cut-off value to obtain the treatment effectiveness of the immunotherapy on the subject having cancer. The second cut-off value is determined by the following steps: a statistical analysis of a correlation between the number of immune cells in a group of subjects having cancer and an expected risk of disease progression in the group of subjects having cancer is performed, and then a statistically significant value used to define the correlation is obtained.

In an embodiment of the invention, the immunotherapy includes anti-PD-1/PD-L1 immunotherapy.

In an embodiment of the invention, the cancer is hepatocellular carcinoma, and the immune cells are selected from the group consisting of PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CD8⁺IFNγ⁺LAG3⁺ cells, PD1⁺CD8⁺IFNγ⁺LAG3⁻ cells, PD1⁺CD8⁺IFNγ⁺TIM3⁺LAG3⁺ cells and PD1⁺CD8⁺IFNγ⁺TIM3⁺LAG3⁻ cells.

In an embodiment of the invention, the cancer is renal cell carcinoma, and the immune cells are selected from the group consisting of PD1⁺CD8⁺ cells, PD1⁺CD8⁺TIM3⁺ cells, PD1⁺CD8⁺IFNγ⁺ cells PD1⁺CD8⁺IFNγ⁺TIM3⁺ cells, PD1⁺CD8⁺IFNγ⁺TIM3⁺LAG3⁻ cells and PD1⁺CD4⁺TGFβ⁺CD25⁺ cells.

In an embodiment of the invention, the cancer is urothelial cancer, and the immune cells are selected from the group consisting of PD1⁺CD8⁺ cells, PD1⁺CD8⁺TIM3⁺ cells, PD1⁺CD8⁺IFNγ⁺ cells, PD1⁺CD8⁺IFNγ⁺TIM3⁺ cells and PD1⁺CD8⁺IFNγ⁺TIM3⁺LAG3⁻ cells.

In an embodiment of the invention, the second cut-off value divided the subject into group A and group B according to a pattern of hazard ratio, wherein the subject classified into group A has a good prognosis, and the subject classified into group B has worse prognosis.

Based on the above, a simple and accurate method for predicting the immunotherapy response of the subject having cancer of the embodiment of the invention is provided, by detecting the number of immune cells in the peripheral blood samples of the subject having cancer and comparing the number of immune cells with the first cut-off value/second cut-off value, it may predict whether the subject having cancer benefits from the immunotherapy before the first treatment, and may obtain treatment effectiveness of the immunotherapy on the subject having cancer soon after the treatment and before the next treatment. Furthermore, compared with conventional technology which is necessary to take tumor cells of subjects having cancer for analysis and thus make the subjects having cancer suffer greater pains, the method provided from the embodiment of the present invention only needs to collect specific immune cells by drawing blood and calculate the number of the immune cells, which may greatly shorten test time and is more convenient for the subjects having cancer. Therefore, the method provided from the embodiments of the present invention has the advantages of saving cost and time for the subjects receiving immunotherapy.

To make the aforementioned more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate exemplary embodiments of the disclosure and, together with the description, serve to explain the principles of the disclosure.
FIG. 1A to FIG. 1G are scatter plots of the number of immune cells of the subject having hepatocellular carcinoma before receiving the first anti-PD1 / PD-L1 immunotherapy and the hazard ratio of the progression free survival (PFS).
FIG. 2A to FIG. 2G are Cox regression models of the progression free survival and the survival probability of the subject having hepatocellular carcinoma after receiving the first anti-PD1 / PD-L1 immunotherapy.
FIG. 3A to FIG. 3C are scatter plots of the number of immune cells of the subject having renal cell carcinoma before receiving the first anti-PD1 / PD-L1 immunotherapy and the hazard ratio of the progression free survival.
FIG. 4A to FIG. 4C are Cox regression models of the progression free survival and the survival probability of the subject having renal cell carcinoma after receiving the first anti-PDl / PD-L1 immunotherapy.
FIG. 5A to FIG. 5I are scatter plots of the number of immune cells of the subject having hepatocellular carcinoma after receiving the anti-PD1 / PD-L1 immunotherapy between the end of one round of treatment until the start of the next round of treatment and the hazard ratio of the progression free survival.
FIG. 6A to FIG. 6I are Cox regression models of the progression free survival and the survival probability of the subject having hepatocellular carcinoma after receiving the anti-PD1 / PD-L1 immunotherapy.
FIG. 7A to FIG. 7F are scatter plots of the number of immune cells of the subject having renal cell carcinoma after receiving the anti-PD1 / PD-L1 immunotherapy between the end of one round of treatment until the start of the next round of treatment and the hazard ratio of the progression free survival.
FIG. 8A to FIG. 8F are Cox regression models of the progression free survival and the survival probability of the subject having renal cell carcinoma after receiving the anti-PD1 / PD-L1 immunotherapy.
FIG. 9A to FIG. 9E are scatter plots of the number of immune cells of the subject having urothelial cancer after receiving the anti-PD1 / PD-L1 immunotherapy between the end of one round of treatment until the start of the next round of treatment and the hazard ratio of the progression free survival.
FIG. 10A to FIG. 10E are Cox regression models of the progression free survival and the survival probability of the subject having urothelial cancer after receiving the anti-PD1 / PD-L1 immunotherapy.

### DESCRIPTION OF THE EMBODIMENTS

### [Definition]

The term "immunotherapy" refers to a method of treating cancer by activating immune system of a cancer patient, such as: immune checkpoint blockade and cellular immunotherapy. According to an embodiment of the present invention, the immunotherapy may refer to administering a PD-1 / PD-L1 inhibitor to a cancer patient to activate the immune system of the cancer patient, thereby achieving the purpose of killing cancer cells. The PD-1 / PD-L1 inhibitor belongs to an immune checkpoint blockade, and the PD-1 / PD-L1 inhibitor may include a PD-1 / PD-L1 antibody, but is not limited thereto.

The term "prognosis" refers to the prediction of the course and outcome of the future development of a disease, particularly cancer. According to the embodiment of the present invention, the prognosis may refer to the progression free survival period of cancer patients. If the progression free survival is longer, it means that the cancer patient has a better prognosis for immunotherapy and a better response to immunotherapy.

For example, in the present embodiment, after the number of immune cells of the evaluated cancer patient is compared with the first cut-off value, if the evaluated cancer patient is classified as Group A with good prognosis, it means that the evaluated cancer patient has a better response to immunotherapy and is suitable for immunotherapy. Conversely, if the evaluated cancer patient is classified as Group B with poor prognosis, it means that the evaluated cancer patient has a worse response to immunotherapy and is not suitable for immunotherapy. In addition, in the present embodiment, after the number of immune cells of the evaluated cancer patient is compared with the second cut-off value, if the evaluated cancer patient is classified as Group A with good prognosis, it means that the evaluated cancer patient has a better response to immunotherapy, the treatment effectiveness can be expected, and the immunotherapy can be continued. Conversely, if the evaluated cancer patient is classified as Group B with poor prognosis, it means that the evaluated cancer patient has a worse response to immunotherapy and is not recommended to continue the immunotherapy.

### [Example 1] A method for predicting immunotherapy response of a subject having cancer who has never received the immunotherapy

In the present embodiment, the cancer may include hepatocellular carcinoma and renal cell carcinoma, but is not limited thereto. The immunotherapy may include immune checkpoint blockade or cellular immunotherapy, but is not limited thereto. The immune checkpoint blockade may include anti-PD-1 / PD-L1 immunotherapy, but is not limited thereto. The following uses the anti-PD-1 / PD-L1 immunotherapy as an example.

The method for predicting anti-PD-1/PD-L1 immunotherapy response of a subject having cancer in the present embodiment may include the following steps. First, step one is proceeded: a peripheral blood sample is obtained from the subject having cancer before receiving the anti-PD1 / PD-L1 immunotherapy. In the present embodiment, a peripheral blood sample (for example 8 mL, but is not limited thereto) is obtained from antecubital veins of the subject having cancer who has never received the anti-PD-1 / PD-L1 immunotherapy. In some embodiments, the peripheral blood sample may also be obtained from veins in other peripheral parts of the subject having cancer.

Next, step two is proceeded: the number of immune cells in the peripheral blood sample of the subject having cancer is detected. In the present embodiment, a pretreatment procedure should be performed on the peripheral blood samples, before detecting the number of immune cells. The pretreatment procedure includes, for example, the following steps, but is not limited thereto: 8 ml of the peripheral blood sample is stained with PE fluorescent dye conjugated anti-PD1 antibody for 20 minutes at room temperature. The stained peripheral blood sample is divided into four 2 ml of blood samples in four 50 ml conical centrifuge tubes. After adding 24 ml of ISOTON II Diluent (Beckman Coulter) into each conical centrifuge tubes, centrifugation is performed at 800 × g for 10 minutes with a swinging bucket rotor at room temperature. After centrifugation, 24 ml of supernatant from each conical centrifuge tubes is removed and four 2 ml of blood samples is respectively mixed into 8 ml of analysis sample. Accordingly, the pretreatment procedure of the peripheral blood samples is completed.

Next, 4 ml of the analysis sample taken out from 8 ml of the analysis sample is first subjected to cell sorting. In the present embodiment, the cell sorting includes, for example, the following steps, but is not limited thereto: MiSelect R System (MiCareo Taiwan Co., Ltd) with SelectChip Dual is used for cell sorting to collect the PD1 Labeled cells (PD1⁺ cells). Then, fixation reagent and antibody mixed reagent A (for example, including CD8-FITC antibody, IFN-γ-PerCP antibody, TIM3-APC antibody, LAG-3 antibody, but is not limited thereto) are automatically added into the collected PD1⁺ cells to further identify cells with PD1 and CD8 markers (PD1⁺CD8⁺ cells) and cells with PD1, CD8 markers and other markers (such as IFN-y, TIM3, LAG3), and then the number of cells are calculated. In the present embodiment, PE, FITC, PerCP and APC are fluorescent dyes that can emit different fluorescent colors.

In addition, after the remaining 4 ml of the 8 ml of the analysis sample is subjected to cell sorting to collect PD1⁺ cells, the fixation reagent and antibody mixing reagent B (for example, including CD4-PerCP antibody, TGF-β-APC Antibody, CD25-FITC antibody, LAG-3 antibody, but is not limited thereto) are automatically added into the collected PD1⁺ cells to further identify cells with PD1 and CD4 markers (PD1⁺CD4⁺ cells) and cells with PD1, CD4 and other markers (such as LAG3, CD25, TGF-β), and then the number of cells are calculated.

Therefore, in the present embodiment, the immune cells may express, for example, at least one of the following markers: PD1, CD8, CD4, IFN-γ, TIM3, LAG3, CD25, TGF-β.For example, the immune cells may include PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CDS⁺IFNγ⁺LAG3⁺ cells, PD1⁺CD8⁺IFNγ⁺TIM3⁺LAG3⁻ cells, PD1⁺CD4⁺TGFβ⁺CD25⁺ cells, PD1⁺CD4⁺TGFβ⁺CD25⁺LAG3⁻ cells and PD1⁺CD4⁺TGFβ⁺CD25⁺LAG3⁺ cells, but is not limited thereto.

Then, step three is proceeded: the number of immune cells is compared with a first cut-off value to indicate whether the subject having cancer benefits from the anti-PD-1 / PD-L1 immunotherapy. In the present embodiment, the first cut-off value is determined by, for example, the following steps, but is not limited thereto: a statistical analysis of a correlation between the number of immune cells in a group of subjects having cancer and an expected risk of disease progression in the group of subjects having cancer is performed, and then a statistically significant value used to define the correlation is obtained. The significant value may be regarded as the first cut-off value. Specifically, the statistically significant value (first cut-off value) may be, for example, the corresponding number of immune cells when the statistical p value is less than or equal to 0.1. Therefore, in the present embodiment, the corresponding number of immune cells (first cut-off value) may be used to define the correlation between the number of immune cells in the subject having cancer and the expected risk of disease progression in the subject having cancer.

In general, the expected risk of disease progression may be a hazard ratio of progression free survival (PFS), but is not limited thereto. Therefore, in the present embodiment, a scatter plot (pattern of hazard ratio) of the number of immune cells of the group of subjects having cancer before receiving anti-PD1 / PD-L1 immunotherapy and the risk ratio of progression free survival may be used to perform the statistical analysis of the correlation to obtain the first cut-off value.

Next, after the number of immune cells of the group of subjects having cancer before receiving the anti-PD1 / PD-L1 immunotherapy is compared with the first cut-off value, the group of subjects having cancer may be divided into group A and group B. Among them, the subjects having cancer classified as group A have good prognosis, that is, the subjects having cancer in the group A have better response to the anti-PD-1 / PD-L1 immunotherapy; the subjects having cancer classified as group B have worse prognosis, that is, the subjects having cancer in the group B have worse response to the PD-1 / PD-L1 immunotherapy.

The relationship between the number of immune cells and the first cut-off value is related to the type of cancer. For example, in the present embodiment, when the number of immune cells of subjects having hepatocellular carcinoma / renal cell carcinoma who have never received the anti-PD-1 / PD-L1 immunotherapy is greater than or equal to the first cut-off value, the subjects having hepatocellular carcinoma / renal cell carcinoma have a good prognosis. However, subjects having other types of cancer may also have a good prognosis when the number of immune cells of the subjects having other types of cancer is less than the first cut-off value.

In the present embodiment, the hazard ratio is measured by, for example, a Cox regression model of a survival time in a group of subjects having cancer versus a survival probability in the group of subjects having cancer. The survival time may be, for example, the progression free survival, but is not limited thereto. In addition, in the Cox regression model, when the survival probability is 50%, it can be found that the progression free survival of the group A with better prognosis may be significantly higher than that of group B with worse prognosis, and statistical p value for this significant difference may be less than or equal to 0.1.

### [Example 2] A method for predicting immunotherapy response of a subject having cancer after receiving the immunotherapy

In the present embodiment, the cancer may include hepatocellular carcinoma, renal cell carcinoma and urothelial cancer, but is not limited thereto. The immunotherapy may include immune checkpoint blockade or cellular immunotherapy, but is not limited thereto. The immune checkpoint blockade may include anti-PD-1 / PD-L1 immunotherapy, but is not limited thereto. The following uses the anti-PD-1 / PD-L1 immunotherapy as an example.

First, step one is proceeded: a peripheral blood sample is obtained from the subject receiving the anti-PD-1/PD-L1 immunotherapy between the end of one round of treatment until the start of the next round of treatment. In the present embodiment, it can be performed according to the steps shown in step one of the above Example 1 and are not repeated in the present embodiment. The main difference between the present embodiment (Example 2) and the Example 1 is that a peripheral blood sample obtained in present embodiment is the peripheral blood sample from the subject receiving the anti-PD-1 / PD-L1 immunotherapy. In addition, since the anti-PD-1 / PD-L1 immunotherapy is usually administered to the subject having cancer every two or three weeks, in the present embodiment, there are, for example, nearly two or three weeks between the end of one round of treatment until the start of the next round of treatment may be used as the timing point to determine treatment effectiveness of the anti-PD-1 / PD-L1 immunotherapy on the subject having cancer, but is not limited thereto.

Next, step two is proceeded: the number of immune cells in the peripheral blood sample of the subject having cancer is detected. In the present embodiment, it can be performed according to the steps shown in step two of the above Example 1 and are not repeated in the present embodiment. The main difference between the present embodiment (Example 2) and the Example 1 is that in the present embodiment the number of immune cells in the peripheral blood sample of the subject receiving the anti-PD-1 / PD-L1 immunotherapy is detected.

Then, step three is proceeded: the number of immune cells is compared with a second cut-off value to obtain the treatment effectiveness of the anti-PD-1/PD-L1immunotherapy on the subject having cancer. In the present embodiment, the second cut-off value may be measured according to the steps shown in step three of the above Example 1 and are not repeated in the present embodiment.

Specifically, in the present embodiment, after the number of immune cells of the subjects having cancer between the end of one round of treatment until the start of the next round of treatment is compared with the second cut-off value, the subjects having cancer are divided into group A' and group B'. Among them, the subjects having cancer classified as group A' have good prognosis, that is, the anti-PD-1 / PD-L1 immunotherapy shows better treatment effectiveness on the subjects having cancer in the group A'; and the subjects having cancer classified as group B' have worse prognosis, that is, the anti-PD-1 / PD-L1 immunotherapy shows worse treatment effectiveness on the subjects having cancer in the group B'.

The relationship between the number of immune cells and the second cut-off value is related to the type of cancer. For example, in one embodiment of the present invention, a subject having hepatocellular carcinoma who has received anti-PD-1 / PD-L1 immunotherapy may have a good prognosis when the number of immune cells is less than the second cut-off value. In another embodiment of the present invention, a subject having renal cell carcinoma and a subject having urothelial cancer who have received anti-PD-1 / PD-L1 immunotherapy may have a good prognosis when the number of immune cells is greater than or equal to the second cut-off value.

### [Experimental]

### Experimental 1: predicting anti-PD-1 / PD-L1 immunotherapy response of subjects having hepatocellular carcinoma who has never received the anti-PD-1 / PD-Llimmunotherapy

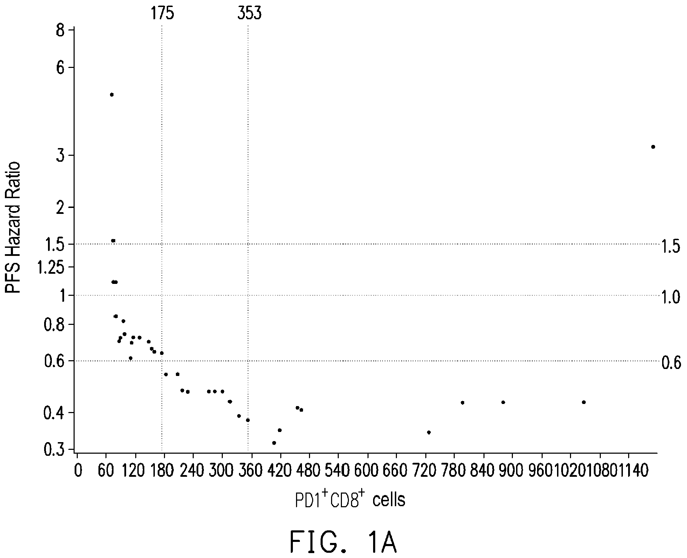

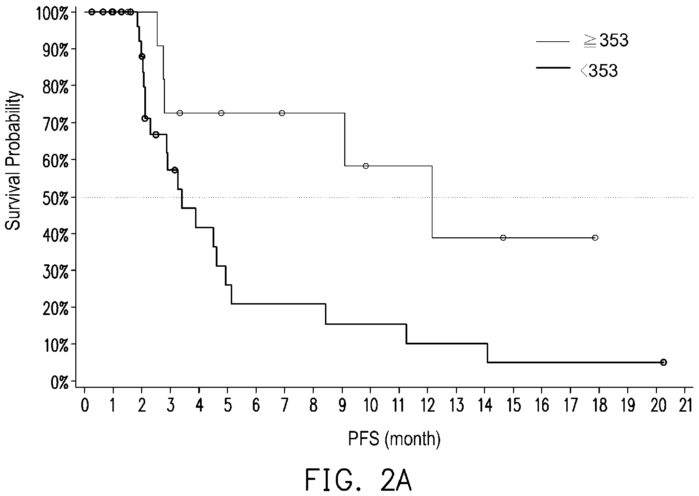

FIG. 1A to FIG. 1G are scatter plots of the number of immune cells of the subject having hepatocellular carcinoma before receiving the first anti-PD1 / PD-L1 immunotherapy and the hazard ratio of the progression free survival (PFS). FIG. 2A to FIG. 2G are Cox regression models of the progression free survival and the survival probability of the subject having hepatocellular carcinoma after receiving the first anti-PD1 / PD-L1 immunotherapy.

Referring to FIGS. 1A and 2A at the same time, in the analysis result of PD1⁺CD8⁺ cells, the first cut-off value is 353. In addition, according to the results of FIG. 2A, when the survival probability is 50%, the progression free survival of the subject having hepatocellular carcinoma who has 353 or more PD1⁺CD8⁺ cells is greater than 12 months, and the progression free survival of the subject having hepatocellular carcinoma who has less than 353 PD1⁺CD8⁺ cells is 3 to 4 months. Here, the statistical p value is 0.0227 and the 95% confidence interval (CI) of the hazard ratio is 0.117 to 0.851.

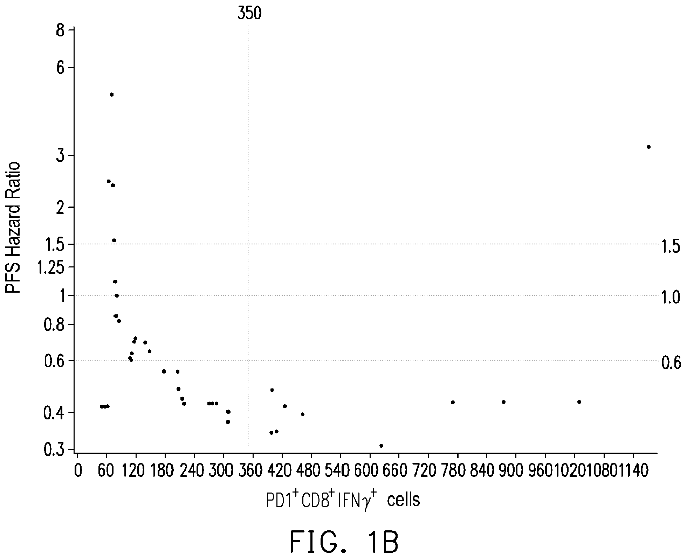

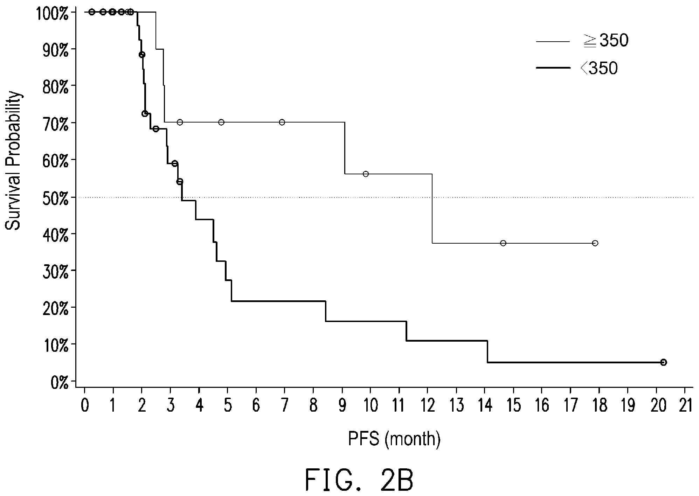

Referring to FIGS. 1B and 2B at the same time, in the analysis result of PD1⁺CD8⁺IFNγ⁺ cells, the first cut-off value is 350. In addition, according to the results of FIG. 2B, when the survival probability is 50%, the progression free survival of the subject having hepatocellular carcinoma who has 350 or more PD1⁺CD8⁺IFNγ⁺ cells is greater than 12 months, and the progression free survival of the subject having hepatocellular carcinoma who has less than 350 PD1⁺CD8⁺IFNγ⁺ cells is 3 to 4 months. Here, the statistical p value is 0.0343 and the 95% confidence interval of the hazard ratio is 0.126 to 0.923.

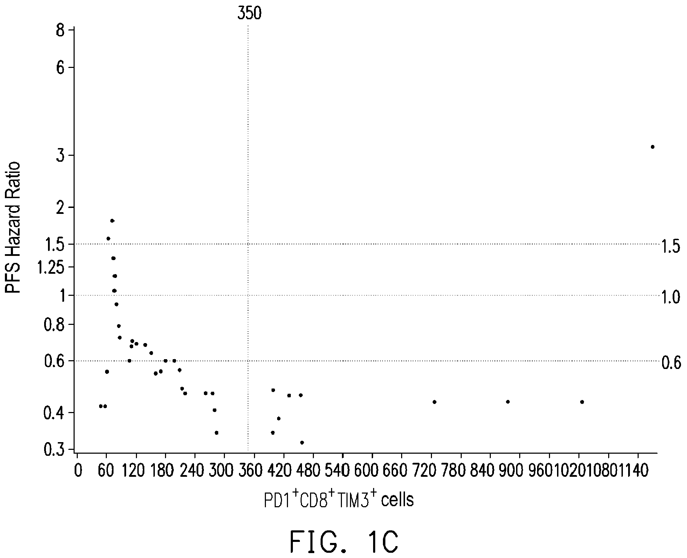

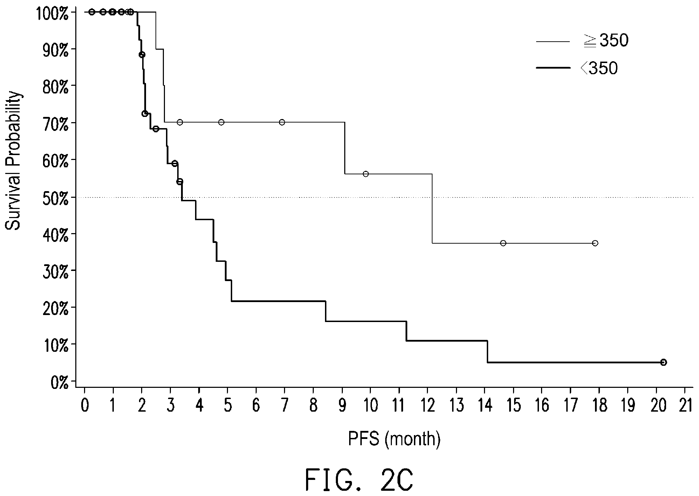

Referring to FIGS. 1C and 2C at the same time, in the analysis result of PD1⁺CD8⁺TIM3⁺ cells, the first cut-off value is 350. In addition, according to the results of FIG. 2C, when the survival probability is 50%, the progression free survival of the subject having hepatocellular carcinoma who has 350 or more PD1⁺CD8⁺TIM3⁺ cells is greater than 12 months, and the progression free survival of the subject having hepatocellular carcinoma who has less than 350 PD1⁺CD8⁺TIM3⁺ cells is 3 to 4 months. Here, the statistical p value is 0.0343 and the 95% confidence interval of the hazard ratio is 0.126 to 0.923.

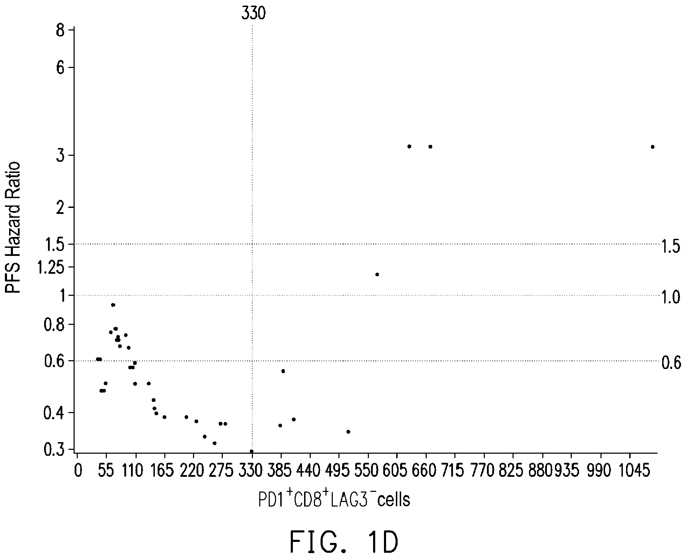

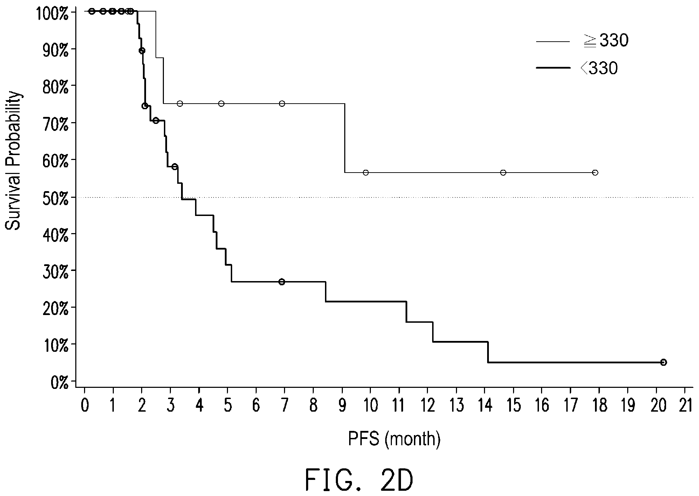

Referring to FIGS. 1D and 2D at the same time, in the analysis result of PD1⁺CD8⁺LAG3⁻ cells, the first cut-off value is 330. In addition, according to the results of FIG. 2D, when the survival probability is 50%, the progression free survival of the subject having hepatocellular carcinoma who has 330 or more PD1⁺CD8⁺LAG3⁻ cells is greater than 18 months, and the progression free survival of the subject having hepatocellular carcinoma who has less than 330 PD1⁺CD8⁺LAG3⁻ cells is 3 to 4 months. Here, the statistical p value is 0.0429 and the 95% confidence interval of the hazard ratio is 0.085 to 0.961.

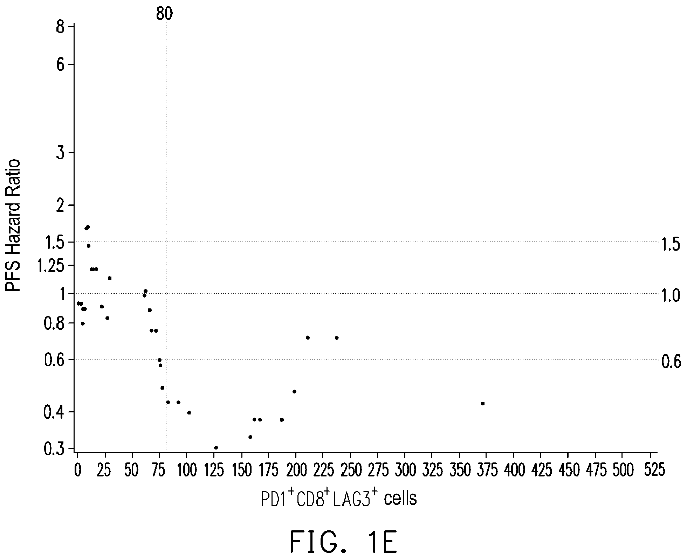

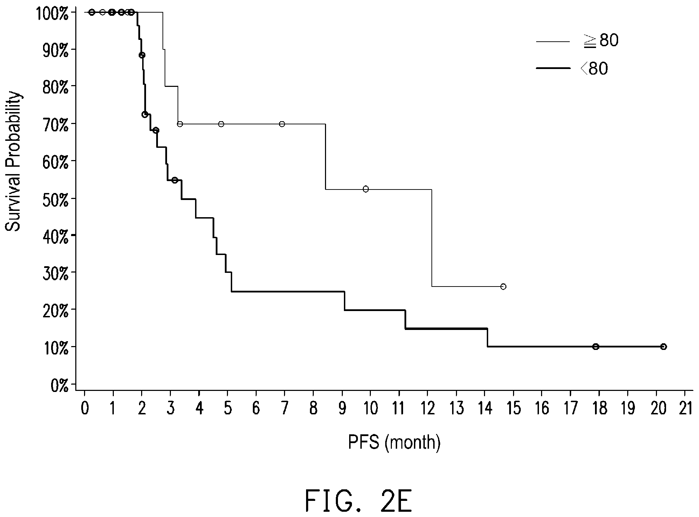

Referring to FIGS. 1E and 2E at the same time, in the analysis result of PD1⁺CD8⁺LAG3⁺ cells, the first cut-off value is 80. In addition, according to the results of FIG. 2E, when the survival probability is 50%, the progression free survival of the subject having hepatocellular carcinoma who has 80 or more PD1⁺CD8⁺LAG3⁺ cells is greater than 12 months, and the progression free survival of the subject having hepatocellular carcinoma who has less than 80 PD1⁺CD8⁺LAG3⁺ cells is 3 to 4 months. Here, the statistical p value is 0.0925 and the 95% confidence interval of the hazard ratio is 0.161 to 1.149.

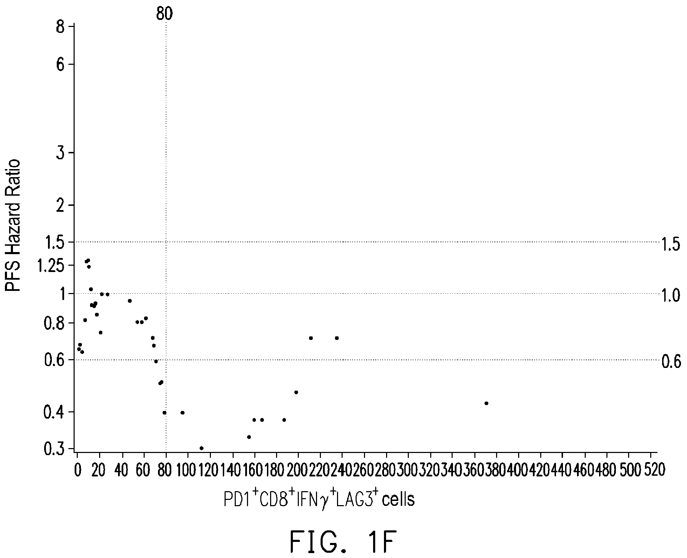

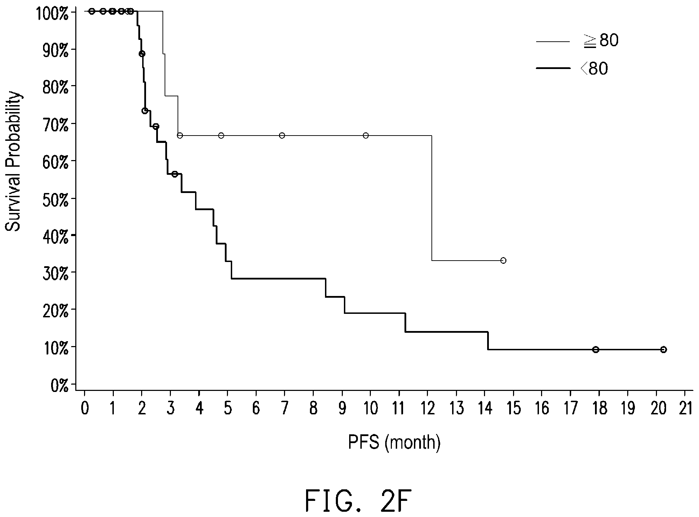

Referring to FIGS. 1F and 2F at the same time, in the analysis result of PD1⁺CD8⁺IFNγ⁺LAG3⁺ cells, the first cut-off value is 80. In addition, according to the results of FIG. 2F, when the survival probability is 50%, the progression free survival of the subject having hepatocellular carcinoma who has 80 or more PD1⁺CD8⁺IFNγ⁺LAG3⁺ cells is greater than 12 months, and the progression free survival of the subject having hepatocellular carcinoma who has less than 80 PD1⁺CD8⁺IFNγ⁺LAG3⁺ cells is 3 to 4 months. Here, the statistical p value is 0.0911 and the 95% confidence interval of the hazard ratio is 0.136 to 1.159.

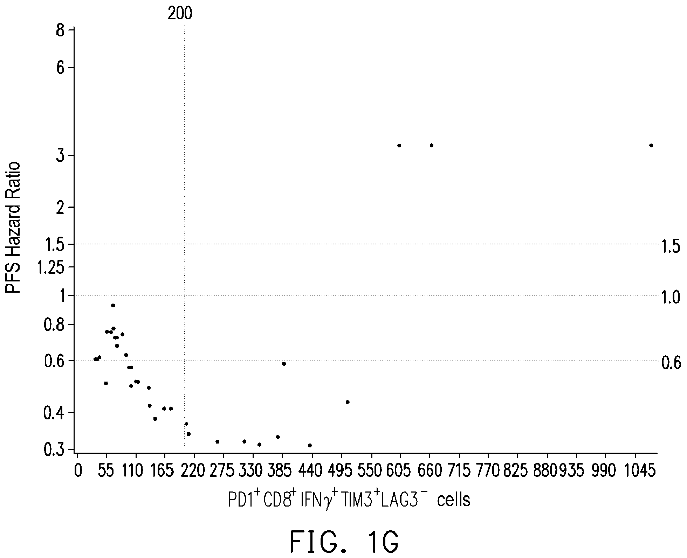

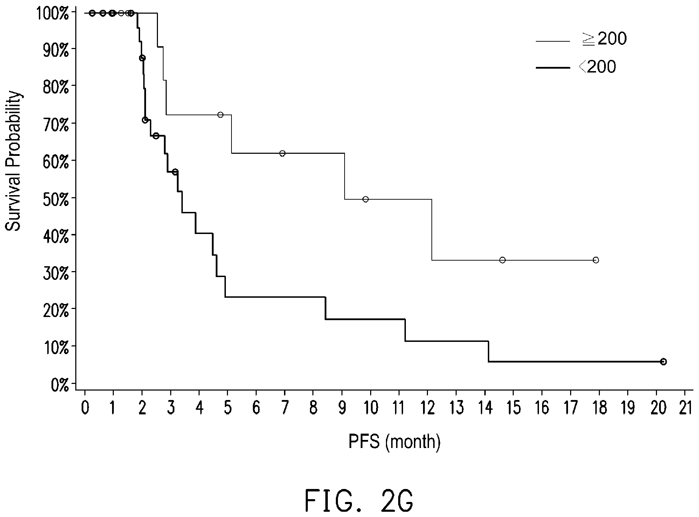

Referring to FIGS. 1G and 2G at the same time, in the analysis result of PD1⁺CD8⁺IFNγ⁺TIM3⁺LAG3⁻ cells, the first cut-off value is 200. In addition, according to the results of FIG. 2G, when the survival probability is 50%, the progression free survival of the subject having hepatocellular carcinoma who has 200 or more PD1⁺CD8⁺IFNγ⁺TIM3⁺LAG3⁻ cells is 9 to 12 months, and the progression free survival of the subject having hepatocellular carcinoma who has less than 200 PD1⁺CD8⁺IFNγ⁺TIM3⁺LAG3⁻ cells is 3 to 4 months. Here, the statistical p value is 0.0353 and the 95% confidence interval of the hazard ratio is 0.145 to 0.933.

Based on the above, according to the results of FIGS. 1A to 1G and FIGS. 2A to 2G, for the subjects having hepatocellular carcinoma who have never received the anti-PD-1 / PD-L1 immunotherapy, when the number of immune cells of the subjects having hepatocellular carcinoma before receiving the first anti-PD1 / PD-L1 immunotherapy is greater than or equal to the first cut-off value, it can be predicted that the subjects having hepatocellular carcinoma have better response to the anti-PD-1 / PD-L1 immunotherapy and have good prognosis. Conversely, when the number of immune cells of the subjects having hepatocellular carcinoma before receiving the anti-PD1 / PD-L1 immunotherapy is less than the first cut-off value, it can be predicted that the subjects having hepatocellular carcinoma have worse response to the anti-PD-1 / PD-L1 immunotherapy and have worse prognosis. Here, the above immune cells include PD1⁺CD8⁺ cells, PD1⁺CD8⁺IFNγ⁺ cells, PD1⁺CD8⁺TIM3⁺ cells, PD1⁺CD8⁺LAG3⁺ cells, PD1⁺CD8⁺LAG3⁻ cells, PD1⁺CD8⁺IFNγ⁺LAG3⁺ Cells, and PD1⁺CD8⁺IFNγ⁺TIM3⁺LAG3⁻ cells, but is not limited thereto.

### Experimental 2: predicting anti-PD-1 / PD-L1 immunotherapy response of subjects having renal cell carcinoma who has never received the anti-PD-1 / PD-Llimmunotherapy

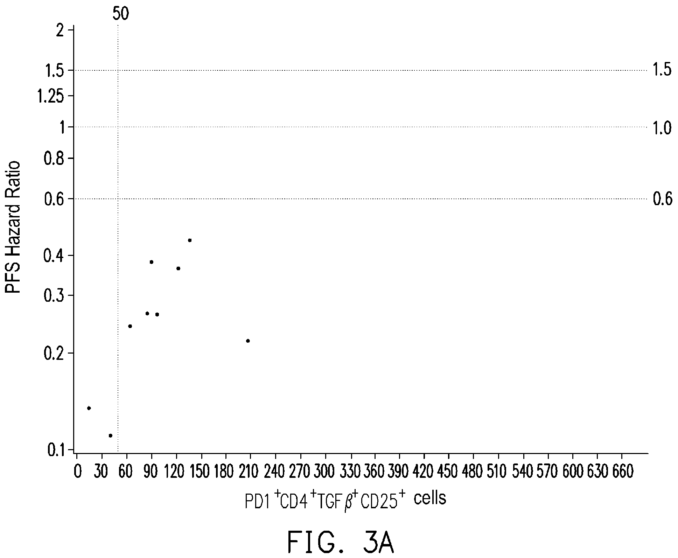

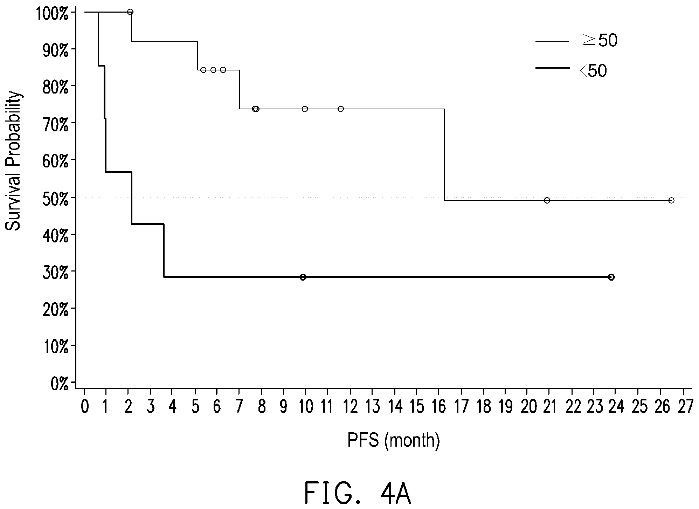

FIG. 3A to FIG. 3C are scatter plots of the number of immune cells of the subject having renal cell carcinoma before receiving the first anti-PD1 / PD-L1 immunotherapy and the hazard ratio of the progression free survival. FIG. 4A to FIG. 4C are Cox regression models of the progression free survival and the survival probability of the subject having renal cell carcinoma after receiving the first anti-PD1 / PD-L1 immunotherapy.

Referring to FIGS. 3A and 4A at the same time, in the analysis result of PD1⁺CD4⁺TGFβ⁺CD25⁺ cells, the first cut-off value is 50. In addition, according to the results of FIG. 4A, when the survival probability is 50%, the progression free survival of the subject having renal cell carcinoma who has 50 or more PD1⁺CD4⁺TGFβ⁺CD25⁺ cells is greater than 16 months, and the progression free survival of the subject having renal cell carcinoma who has less than 50 PD1⁺CD4⁺TGFβ⁺CD25⁺ cells is 2 to 3 months. Here, the statistical p value is 0.0369 and the 95% confidence interval of the hazard ratio is 0.064 to 0.917.

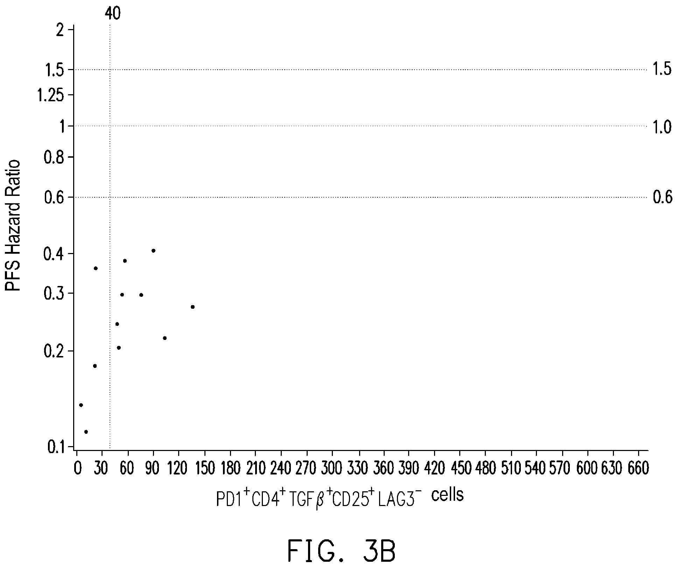

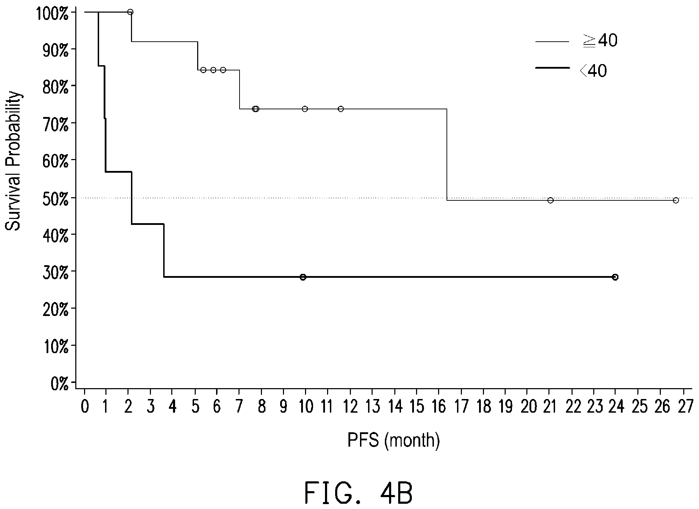

Referring to FIGS. 3B and 4B at the same time, in the analysis result of PD1⁺CD4⁺TGFβ⁺CD25⁺LAG3⁻ cells, the first cut-off value is 40. In addition, according to the results of FIG. 4B, when the survival probability is 50%, the progression free survival of the subject having renal cell carcinoma who has 40 or more PD1⁺CD4⁺TGFβ⁺CD25⁺LAG3⁻ cells is greater than 16 months, and the progression free survival of the subject having renal cell carcinoma who has less than 40 PD1⁺CD4⁺TGFβ⁺CD25⁺LAG3⁻ cells is 2 to 3 months. Here, the statistical p value is 0.0369 and the 95% confidence interval of the hazard ratio is 0.064 to 0.917.

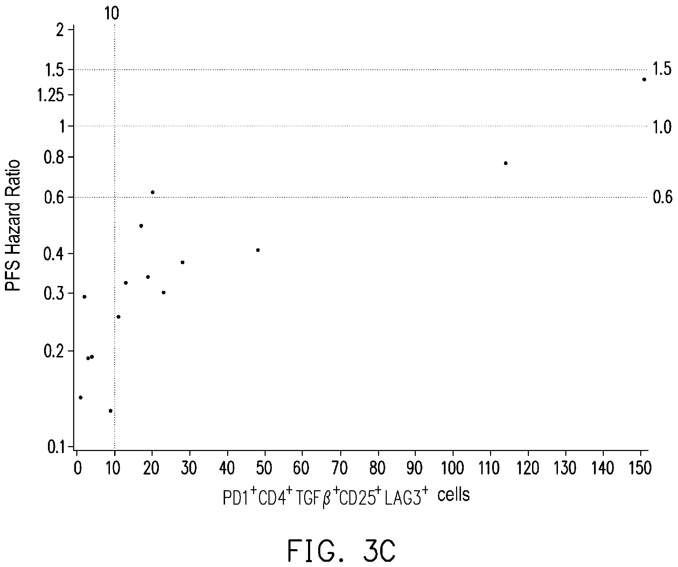

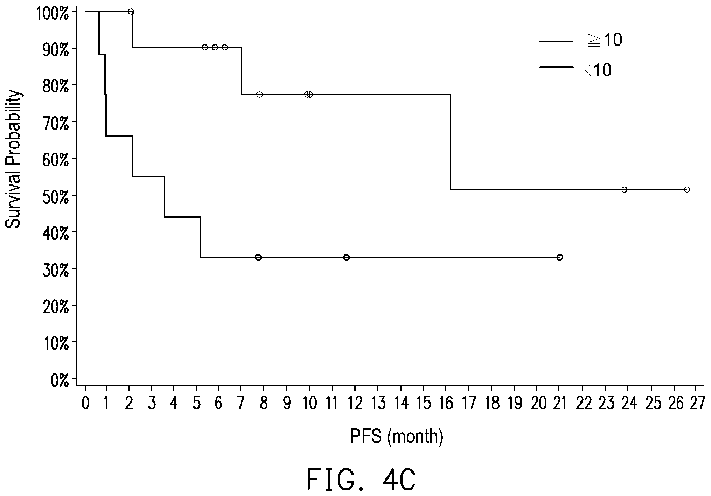

Referring to FIGS. 3C and 4C at the same time, in the analysis result of PD1⁺CD4⁺TGFβ⁺CD25⁺LAG3⁺ cells, the first cut-off value is 10. In addition, according to the results of FIG. 4C, when the survival probability is 50%, the progression free survival of the subject having renal cell carcinoma who has 10 or more PD1⁺CD4⁺TGFβ⁺CD25⁺LAG3⁺ cells is greater than 16 months, and the progression free survival of the subject having renal cell carcinoma who has less than 10 PD1⁺CD4⁺TGFβ⁺CD25⁺LAG3⁺ cells is 3 to 4 months. Here, the statistical p value is 0.0534 and the 95% confidence interval of the hazard ratio is 0.063 to 1.021.

Based on the above, according to the results of FIGS. 3A to 3C and FIGS. 4A to 4C, for the subjects having renal cell carcinoma who have never received the first anti-PD-1 / PD-L1 immunotherapy, when the number of immune cells of the subjects having renal cell carcinoma before receiving the anti-PD1 / PD-L1 immunotherapy is greater than or equal to the first cut-off value, it can be predicted that the subjects having renal cell carcinoma have better response to the anti-PD-1 / PD-L1 immunotherapy and have good prognosis. Conversely, when the number of immune cells of the subjects having renal cell carcinoma before receiving the anti-PD1 / PD-L1 immunotherapy is less than the first cut-off value, it can be predicted that the subjects having renal cell carcinoma have worse response to the anti-PD-1 / PD-L1 immunotherapy and have worse prognosis. Here, the above immune cells include PD1⁺CD4⁺TGFβ⁺CD25⁺ cells, PD1⁺CD4⁺TGFβ⁺CD25⁺LAG3⁻ cells, and PD1⁺CD4⁺TGFβ⁺CD25⁺LAG3⁺ cells, but is not limited thereto.

### Experimental 3: predicting anti-PD-1 / PD-L1 immunotherapy response of subjects having hepatocellular carcinoma after receiving the anti-PD-1 / PD-Llimmunotherapy

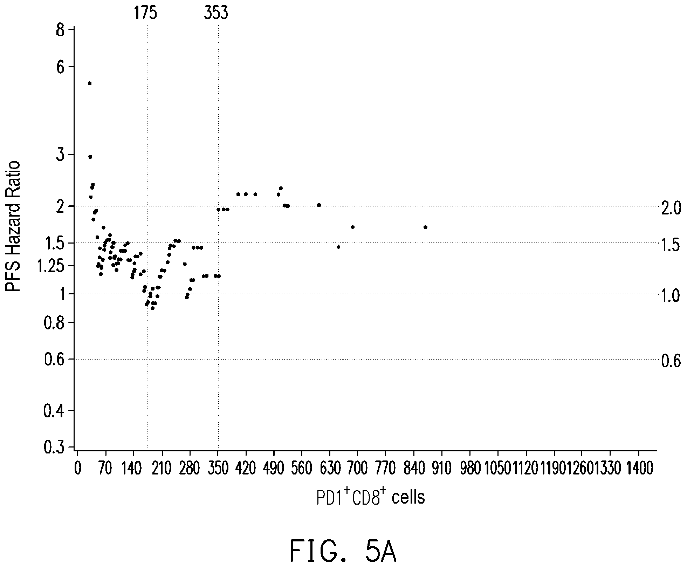

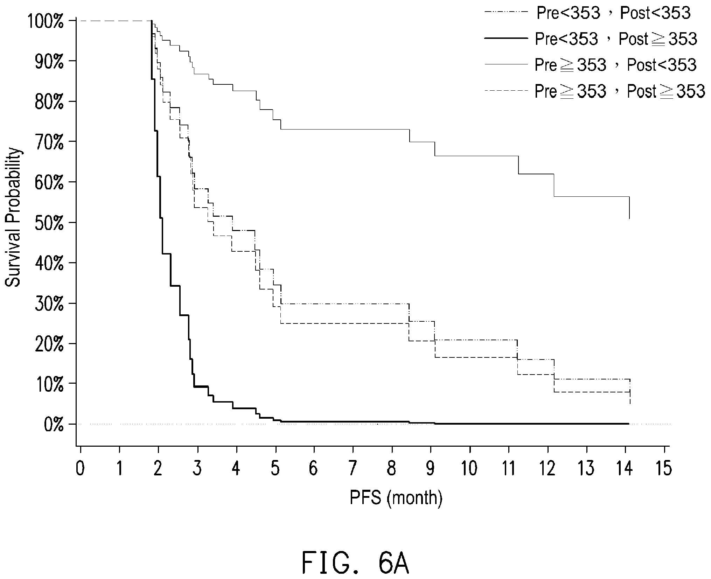

FIG. 5A to FIG. 5I are scatter plots of the number of immune cells of the subject having hepatocellular carcinoma after receiving the anti-PD1 / PD-L1 immunotherapy between the end of one round of treatment until the start of the next round of treatment and the hazard ratio of the progression free survival. FIG. 6A to FIG. 6I are Cox regression models of the progression free survival and the survival probability of the subject having hepatocellular carcinoma after receiving the anti-PDl / PD-L1 immunotherapy.

Referring to FIGS. 5A and 6A at the same time, in the analysis results of PD1⁺CD8⁺ cells, the first cut-off value is 353 and the second cut-off value is 353. In addition, according to the results of FIG. 6A, when the survival probability is 50%, the progression free survival of the subject having hepatocellular carcinoma who has 353 or more PD1⁺CD8⁺ cells before treatment (Pre) and has less than 353 PD1⁺CD8⁺ cells after treatment (Post) is greater than 14 months; the progression free survival of the subject having hepatocellular carcinoma who has 353 or more PD1⁺CD8⁺ cells before treatment (Pre) and has 353 or more PD1⁺CD8⁺ cells after treatment (Post) is 3 to 4 months; the progression free survival of the subject having hepatocellular carcinoma who has less than 353 PD1⁺CD8⁺ cells before treatment (Pre) and has less than 353 PD1⁺CD8⁺ cells after treatment (Post) is 4 to 5 months; the progression free survival of the subject having hepatocellular carcinoma who has less than 353 PD1⁺CD8⁺ cells before treatment (Pre) and has 353 or more PD1⁺CD8⁺ cells after treatment (Post) is 2 to 3 months. Here, the statistical p-value is 0.0480 and the 95% confidence interval of the hazard ratio is 1.013 to 19.012.

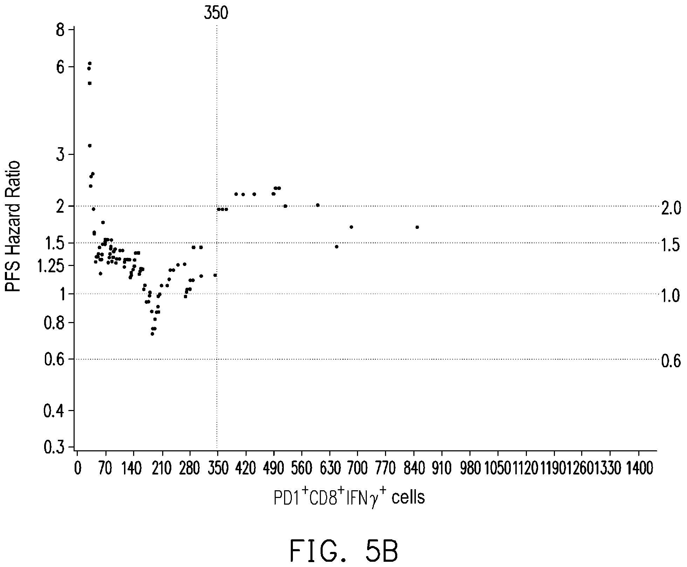

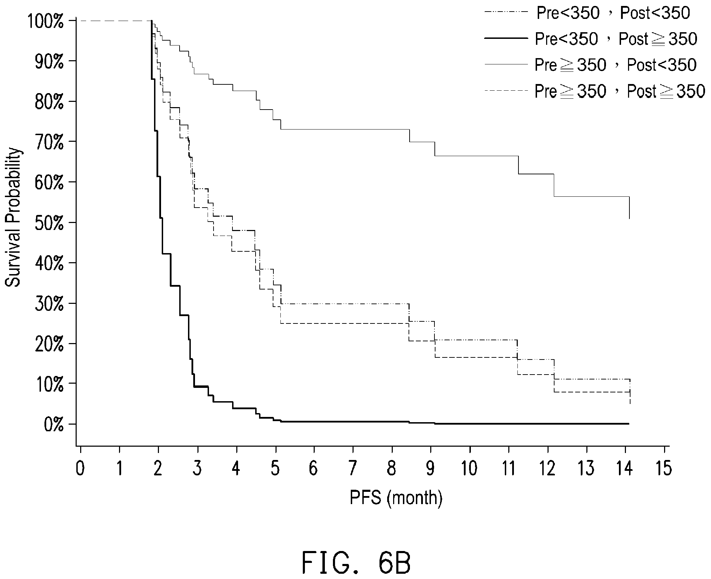

Referring to FIGS. 5B and 6B at the same time, in the analysis results of PD1 ⁺CD8⁺IFNγ⁺ cells, the first cut-off value is 350 and the second cut-off value is 350. In addition, according to the results of FIG. 6B, when the survival probability is 50%, the progression free survival of the subject having hepatocellular carcinoma who has 350 or more PD1⁺CD8⁺IFNγ⁺ cells before treatment (Pre) and has less than 350 PD1⁺CD8⁺IFNγ⁺ cells after treatment (Post) is greater than 14 months; the progression free survival of the subject having hepatocellular carcinoma who has 350 or more PD1⁺CD8⁺IFNγ⁺ cells before treatment (Pre) and has 350 or more PD1⁺CD8⁺IFNγ⁺ cells after treatment (Post) is 3 to 4 months; the progression free survival of the subject having hepatocellular carcinoma who has less than 350 PD1⁺CD8⁺IFNγ⁺ cells before treatment (Pre) and has less than 350 PD1⁺CD8⁺IFNγ⁺ cells after treatment (Post) is 3 to 4 months; the progression free survival of the subject having hepatocellular carcinoma who has less than 350 PD1⁺CD8⁺IFNγ⁺ cells before treatment (Pre) and has 350 or more PD1⁺CD8⁺IFNγ⁺ cells after treatment (Post) is 2 to 3 months. Here, the statistical p-value is 0.0480 and the 95% confidence interval of the hazard ratio is 1.013 to 19.012.

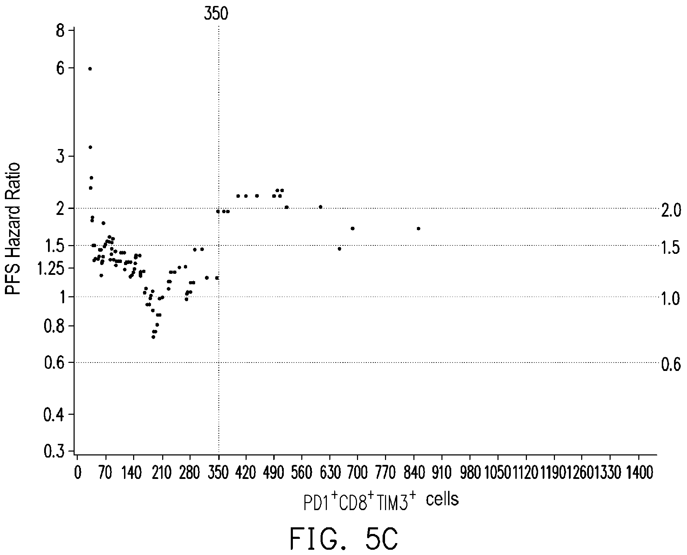

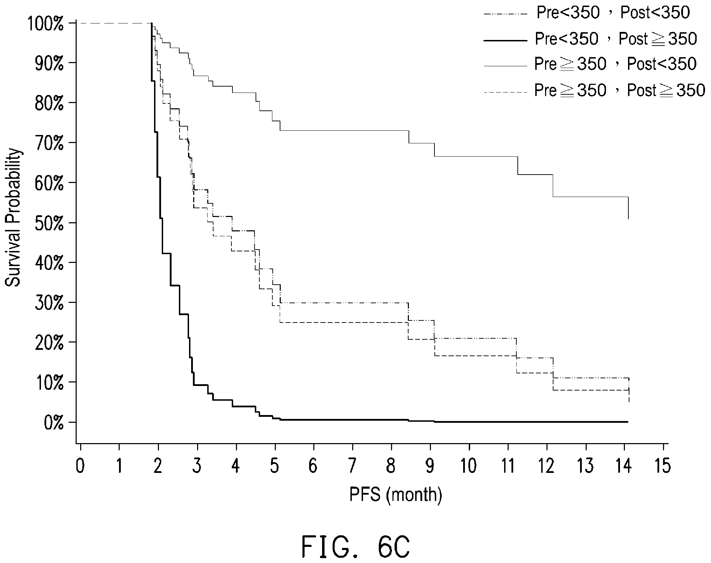

Referring to FIGS. 5C and 6C at the same time, in the analysis results of PD1⁺CD8⁺TIM3⁺ cells, the first cut-off value is 350 and the second cut-off value is 350. In addition, according to the results of FIG. 6C, when the survival probability is 50%, the progression free survival of the subject having hepatocellular carcinoma who has 350 or more PD1⁺CD8⁺TIM3⁺ cells before treatment (Pre) and has less than 350 PD1⁺CD8⁺TIM3⁺ cells after treatment (Post) is greater than 14 months; the progression free survival of the subject having hepatocellular carcinoma who has 350 or more PD1⁺CD8⁺TIM3⁺ cells before treatment (Pre) and has 350 or more PD1⁺CD8⁺TIM3⁺ cells after treatment (Post) is 3 to 4 months; the progression free survival of the subject having hepatocellular carcinoma who has less than 350 PD1⁺CD8⁺TIM3⁺ cells before treatment (Pre) and has less than 350 PD1⁺CD8⁺TIM3⁺ cells after treatment (Post) is 3 to 4 months; the progression free survival of the subject having hepatocellular carcinoma who has less than 350 PD1⁺CD8⁺TIM3⁺ cells before treatment (Pre) and has 350 or more PD1⁺CD8⁺TIM3⁺ cells after treatment (Post) is 2 to 3 months. Here, the statistical p-value is 0.0480 and the 95% confidence interval of the hazard ratio is 1.013 to 19.012.

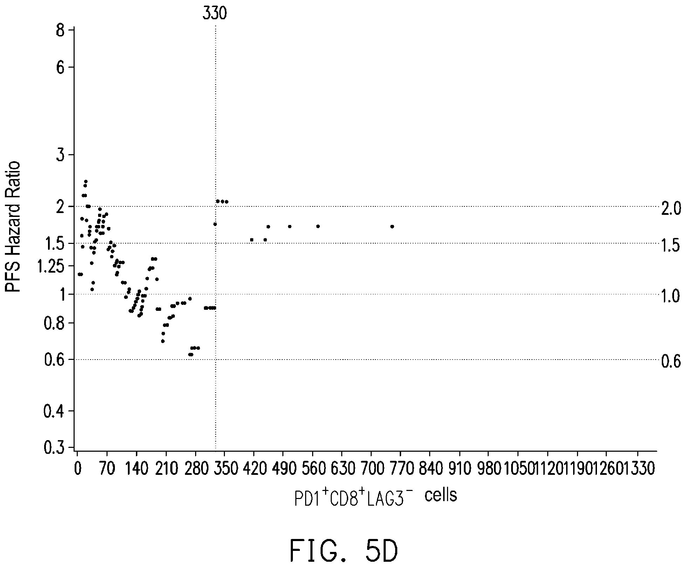

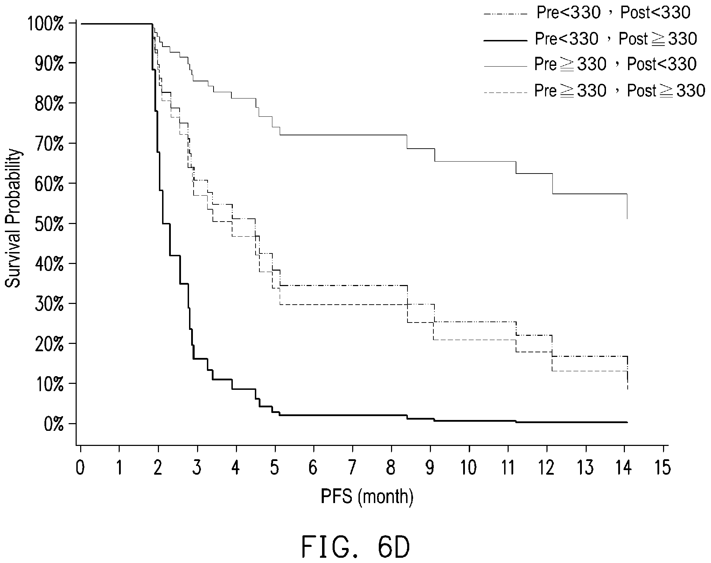

Referring to FIGS. 5D and 6D at the same time, in the analysis results of PD1⁺CD8⁺LAG3⁻ cells, the first cut-off value is 330 and the second cut-off value is 330. In addition, according to the results of FIG. 6D, when the survival probability is 50%, the progression free survival of the subject having hepatocellular carcinoma who has 330 or more PD1⁺CD8⁺LAG3⁻ cells before treatment (Pre) and has less than 330 PD1⁺CD8⁺LAG3⁻ cells after treatment (Post) is greater than 14 months; the progression free survival of the subject having hepatocellular carcinoma who has 330 or more PD1⁺CD8⁺LAG3⁻ cells before treatment (Pre) and has 330 or more PD1⁺CD8⁺LAG3⁻ cells after treatment (Post) is 3 to 4 months; the progression free survival of the subject having hepatocellular carcinoma who has less than 330 PD1⁺CD8⁺LAG3⁻ cells before treatment (Pre) and has less than 330 PD1⁺CD8⁺LAG3⁻ cells after treatment (Post) is 4 to 5 months; the progression free survival of the subject having hepatocellular carcinoma who has less than 330 PD1⁺CD8⁺LAG3⁻ cells before treatment (Pre) and has 330 or more PD1⁺CDS⁺LAG3⁻ cells after treatment (Post) is 2 to 3 months. Here, the statistical p-value is 0.0602 and the 95% confidence interval of the hazard ratio is 0.946 to 14.350.

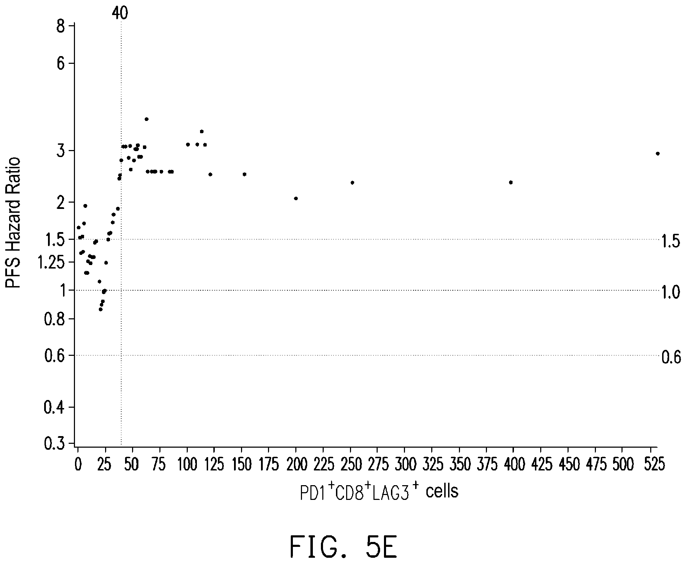

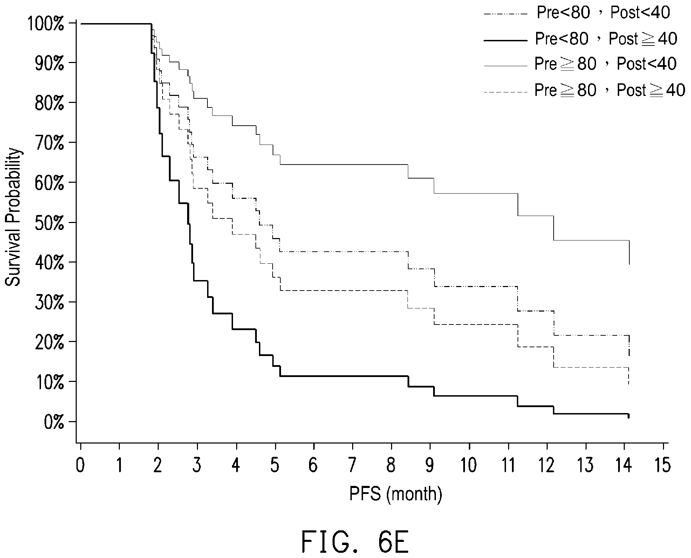

Referring to FIGS. 5E and 6E at the same time, in the analysis results of PD1⁺CD8⁺LAG3⁺ cells, the first cut-off value is 80 and the second cut-off value is 40. In addition, according to the results of FIG. 6E, when the survival probability is 50%, the progression free survival of the subject having hepatocellular carcinoma who has 80 or more PD1⁺CD8⁺LAG3⁺ cells before treatment (Pre) and has less than 40 PD1⁺CD8⁺LAG3⁺ cells after treatment (Post) is greater than 12 months; the progression free survival of the subject having hepatocellular carcinoma who has 80 or more PD1⁺CD8⁺LAG3⁺ cells before treatment (Pre) and has 40 or more PD1⁺CD8⁺LAG3⁺ cells after treatment (Post) is 3 to 4 months; the progression free survival of the subject having hepatocellular carcinoma who has less than 80 PD1⁺CD8⁺LAG3⁺ cells before treatment (Pre) and has less than 40 PD1⁺CD8⁺LAG3⁺ cells after treatment (Post) is 4 to 5 months; the progression free survival of the subject having hepatocellular carcinoma who has less than 80 PD1⁺CD8⁺LAG3⁺ cells before treatment (Pre) and has 40 or more PD1⁺CD8⁺LAG3⁺ cells after treatment (Post) is 2 to 3 months. Here, the statistical p-value is 0.0454 and the 95% confidence interval of the hazard ratio is 1.019 to 6.342.

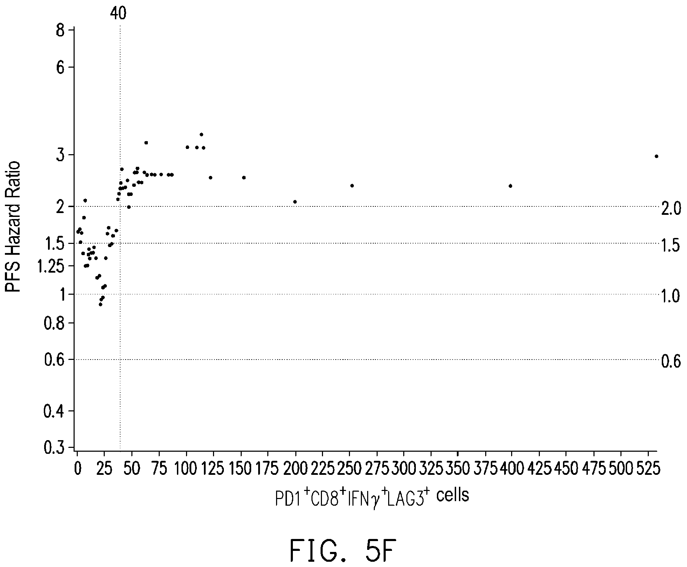

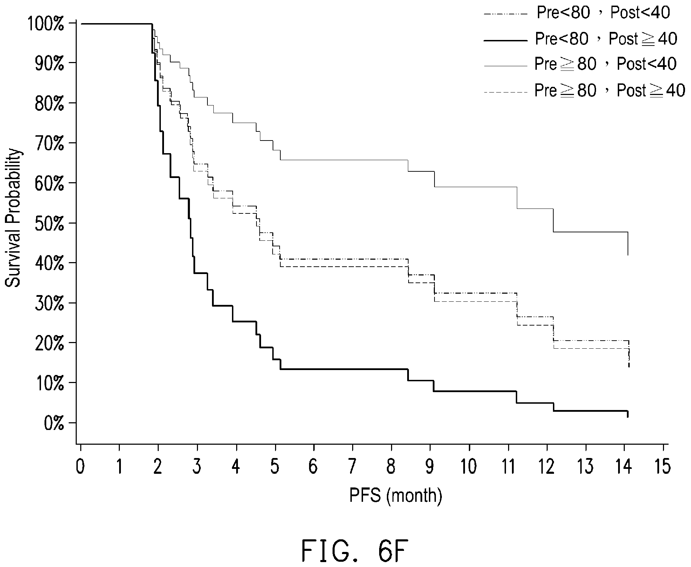

Referring to FIGS. 5F and 6F at the same time, in the analysis results of PD1⁺CD8⁺IFNγ⁺LAG3⁺ cells, the first cut-off value is 80 and the second cut-off value is 40. In addition, according to the results of FIG. 6E, when the survival probability is 50%, the progression free survival of the subject having hepatocellular carcinoma who has 80 or more PD1⁺CD8⁺IFNγ⁺LAG3⁺ cells before treatment (Pre) and has less than 40 PD1⁺CD8⁺IFNγ⁺LAG3⁺ cells after treatment (Post) is greater than 12 months; the progression free survival of the subject having hepatocellular carcinoma who has 80 or more PD1⁺CD8⁺IFNγ⁺LAG3⁺ cells before treatment (Pre) and has 40 or more PD1⁺CD8⁺IFNγ⁺LAG3⁺ cells after treatment (Post) is 4 to 5 months; the progression free survival of the subject having hepatocellular carcinoma who has less than 80 PD1⁺CD8⁺IFNγ⁺LAG3⁺ cells before treatment (Pre) and has less than 40 PD1⁺CD8⁺IFNγ⁺LAG3⁺ cells after treatment (Post) is 4 to 5 months; the progression free survival of the subject having hepatocellular carcinoma who has less than 80 PD1⁺CDS⁺IFNγ⁺LAG3⁺ cells before treatment (Pre) and has 40 or more PD1⁺CD8⁺IFNγ⁺LAG3⁺ cells after treatment (Post) is 2 to 3 months. Here, the statistical p-value is 0.0930 and the 95% confidence interval of the hazard ratio is 0.872 to 5.899.

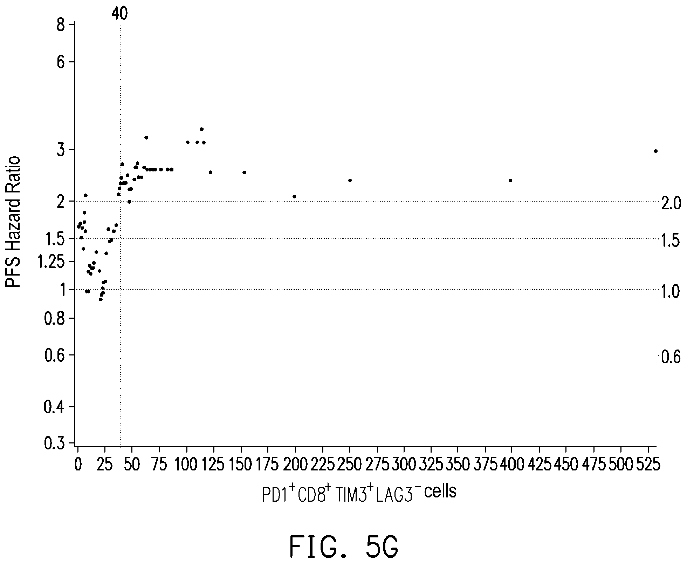

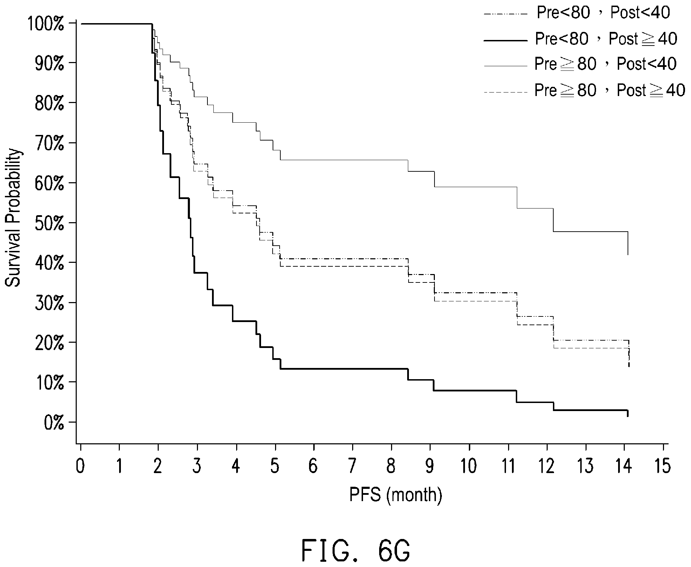

Referring to FIGS. 5G and 6G at the same time, in the analysis results of PD1⁺CD8⁺TIM3⁺LAG3⁻ cells, the first cut-off value is 80 and the second cut-off value is 40. In addition, according to the results of FIG. 6G, when the survival probability is 50%, the progression free survival of the subject having hepatocellular carcinoma who has 80 or more PD1⁺CD8⁺TIM3⁺LAG3⁻ cells before treatment (Pre) and has less than 40 PD1⁺CD8⁺TIM3⁺LAG3⁻ cells after treatment (Post) is greater than 12 months; the progression free survival of the subject having hepatocellular carcinoma who has 80 or more PD1⁺CD8⁺TIM3⁺LAG3⁻ cells before treatment (Pre) and has 40 or more PD1⁺CD8⁺TIM3⁺LAG3⁻ cells after treatment (Post) is 4 to 5 months; the progression free survival of the subject having hepatocellular carcinoma who has less than 80 PD1⁺CD8⁺TIM3⁺LAG3⁻ cells before treatment (Pre) and has less than 40 PD1⁺CD8⁺TIM3⁺LAG3⁻ cells after treatment (Post) is 4 to 5 months; the progression free survival of the subject having hepatocellular carcinoma who has less than 80 PD1⁺CD8⁺TIM3⁺LAG3⁻ cells before treatment (Pre) and has 40 or more PD1⁺CD8⁺TIM3⁺LAG3⁻ cells after treatment (Post) is 2 to 3 months. Here, the statistical p-value is 0.0930 and the 95% confidence interval of the hazard ratio is 0.872 to 5.899.

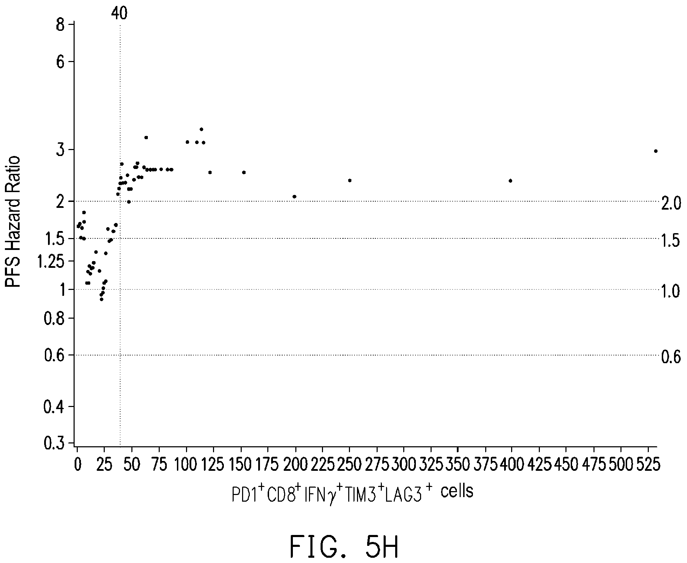

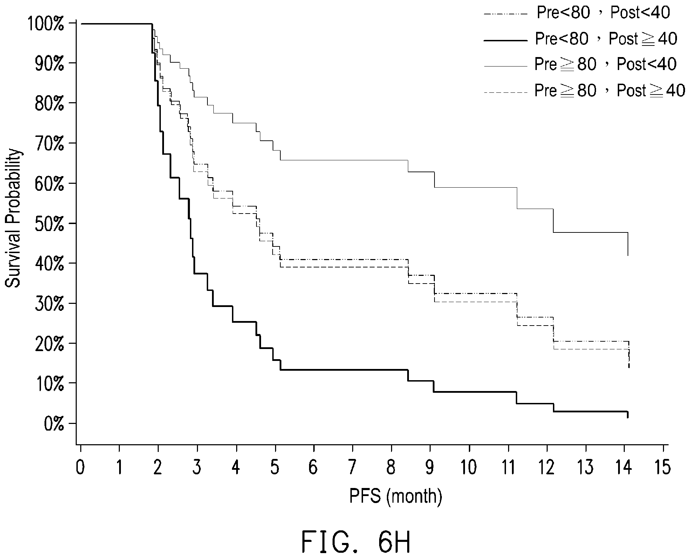

Referring to FIGS. 5H and 6H at the same time, in the analysis results of PD1⁺CD8⁺IFNγ⁺TIM3⁺LAG3⁺ cells, the first cut-off value is 80 and the second cut-off value is 40. In addition, according to the results of FIG. 6H, when the survival probability is 50%, the progression free survival of the subject having hepatocellular carcinoma who has 80 or more PD1⁺CD8⁺IFNγ⁺TIM3⁺LAG3⁺ cells before treatment (Pre) and has less than 40 PD1⁺CD8⁺IFNγ⁺TIM3⁺LAG3⁺ cells after treatment (Post) is greater than 12 months; the progression free survival of the subject having hepatocellular carcinoma who has 80 or more PD1⁺CD8⁺IFNγ⁺TIM3⁺LAG3⁺ cells before treatment (Pre) and has 40 or more PD1⁺CD8⁺IFNγ⁺TIM3⁺LAG3⁺ cells after treatment (Post) is 4 to 5 months; the progression free survival of the subject having hepatocellular carcinoma who has less than 80 PD1⁺CD8⁺IFNγ⁺TIM3⁺LAG3⁺ cells before treatment (Pre) and has less than 40 PD1⁺CD8⁺IFNγ⁺TIM3⁺LAG3⁺ cells after treatment (Post) is 4 to 5 months; the progression free survival of the subject having hepatocellular carcinoma who has less than 80 PD1⁺CD8⁺IFNγ⁺TIM3⁺LAG3⁺ cells before treatment (Pre) and has 40 or more PD1⁺CD8⁺IFNγ⁺TIM3⁺LAG3⁺ cells after treatment (Post) is 2 to 3 months. Here, the statistical p-value is 0.0930 and the 95% confidence interval of the hazard ratio is 0.872 to 5.899.

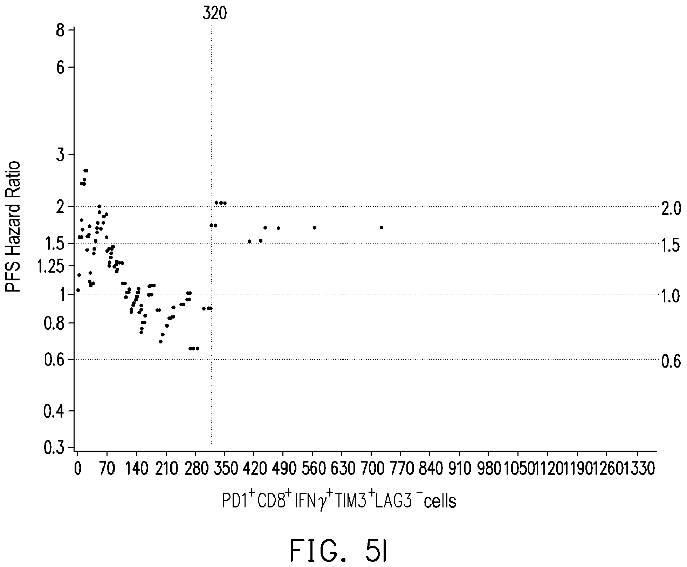

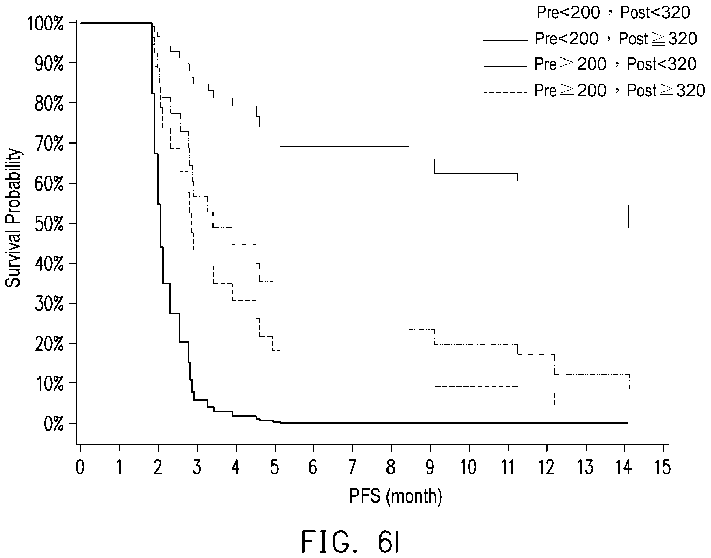

Referring to FIGS. 5I and 6I at the same time, in the analysis results of PD1⁺CDS⁺IFNγ⁺TIM3⁺LAG3⁻ cells, the first cut-off value is 200 and the second cut-off value is 320. In addition, according to the results of FIG. 6I, when the survival probability is 50%, the progression free survival of the subject having hepatocellular carcinoma who has 200 or more PD1⁺CD8⁺IFNγ⁺TIM3⁺LAG3⁻ cells before treatment (Pre) and has less than 320 PD1⁺CD8⁺IFNγ⁺TIM3⁺LAG3⁻ cells after treatment (Post) is greater than 14 months; the progression free survival of the subject having hepatocellular carcinoma who has 200 or more PD1⁺CD8⁺IFNγ⁺TIM3⁺LAG3⁻ cells before treatment (Pre) and has 320 or more PD1⁺CD8⁺IFNγ⁺TIM3⁺LAG3⁻ cells after treatment (Post) is 3 to 4 months; the progression free survival of the subject having hepatocellular carcinoma who has less than 200 PD1⁺CD8⁺IFNγ⁺TIM3⁺LAG3⁻ cells before treatment (Pre) and has less than 320 PD1⁺CD8⁺IFNγ⁺TIM3⁺LAG3⁻ cells after treatment (Post) is 3 to 4 months; the progression free survival of the subject having hepatocellular carcinoma who has less than 200 PD1⁺CD8⁺IFNγ⁺TIM3⁺LAG3⁻ cells before treatment (Pre) and has 320 or more PD1⁺CD8⁺IFNγ⁺TIM3⁺LAG3⁻ cells after treatment (Post) is 2 to 3 months. Here, the statistical p-value is 0.0381 and the 95% confidence interval of the hazard ratio is 1.093 to 23.386.

Based on the above, according to the results of FIGS. 5A to 5I and FIGS. 6A to 6I, for the subjects having hepatocellular carcinoma who have received the anti-PD-1 / PD-L1 immunotherapy, when the number of immune cells of the subjects having hepatocellular carcinoma is greater than or equal to the first cut-off value before treatment (Pre) and is less than the second cut-off value after treatment (Post), the subjects having hepatocellular carcinoma have good prognosis and the anti-PD-1 / PD-L1 immunotherapy shows better treatment effectiveness on the subjects having hepatocellular carcinoma. However, for the subjects having hepatocellular carcinoma who have received the anti-PD-1/PD-L1 immunotherapy, when the number of immune cells of the subjects having hepatocellular carcinoma is greater than or equal to the first cut-off value before treatment (Pre) and is greater than or equal to the second cut-off value after treatment (Post), when the number of immune cells of the subjects having hepatocellular carcinoma is less than the first cut-off value before treatment (Pre) and is less than the second cut-off value after treatment (Post), or when the number of immune cells of the subjects having hepatocellular carcinoma is greater than or equal to the first cut-off value before treatment (Pre) and is less than the second cut-off value after treatment (Post), the subjects having hepatocellular carcinoma have worse prognosis and the anti-PD-1 / PD-L1 immunotherapy shows worse treatment effectiveness on the subjects having hepatocellular carcinoma. Here, the above immune cells include PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CD8⁺IFNγ⁺LAG3⁺ cells, PD1⁺CD8⁺TIM3⁺LAG3⁻ cells, PD1⁺CD8⁺IFNγ⁺TIM3⁺LAG3⁺ cells, and PD1⁺CD8⁺IFNγ⁺TIM3⁺LAG3⁻ cells, but is not limited thereto.

### Experimental 4: predicting anti-PD-1 / PD-L1 immunotherapy response of subjects having renal cell carcinoma after receiving the anti-PD-1 / PD-Llimmunotherapy

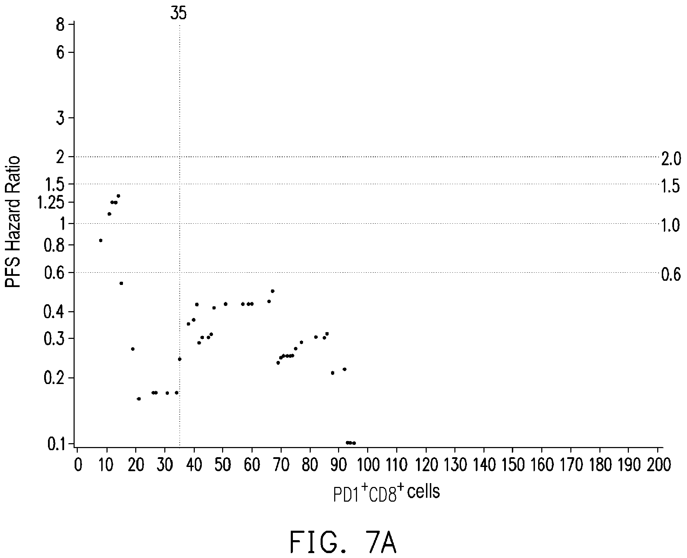

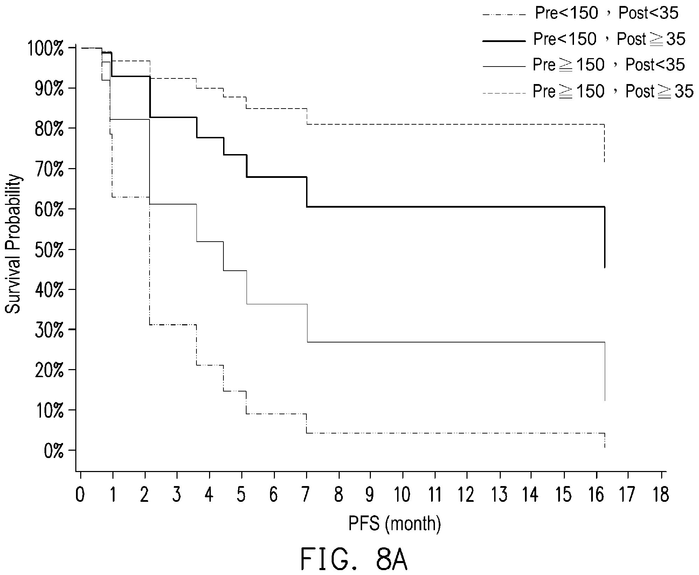

FIG. 7A to FIG. 7F are scatter plots of the number of immune cells of the subject having renal cell carcinoma after receiving the anti-PD1/ PD-L1 immunotherapy between the end of one round of treatment until the start of the next round of treatment and the hazard ratio of the progression free survival. FIG. 8A to FIG. 8F are Cox regression models of the progression free survival and the survival probability of the subject having renal cell carcinoma after receiving the anti-PD 1 / PD-L1 immunotherapy.

Referring to FIGS. 7A and 8A at the same time, in the analysis results of PD1⁺CD8⁺ cells, the first cut-off value is 150 and the second cut-off value is 35. In addition, according to the results of FIG. 8A, when the survival probability is 50%, the progression free survival of the subject having renal cell carcinoma who has 150 or more PD1⁺CD8⁺ cells before treatment (Pre) and has 35 or more PD1⁺CD8⁺ cells after treatment (Post) is greater than 16 months; the progression free survival of the subject having renal cell carcinoma who has less than 150 PD1⁺CD8⁺ cells before treatment (Pre) and has 35 or more PD1⁺CD8⁺ cells after treatment (Post) is greater than 16 months; the progression free survival of the subject having renal cell carcinoma who has 150 or more PD1⁺CD8⁺ cells before treatment (Pre) and has less than 35 PD1⁺CD8⁺ cells after treatment (Post) is 4 to 5 months; the progression free survival of the subject having renal cell carcinoma who has less than 150 PD1⁺CD8⁺ cells before treatment (Pre) and has less than 35 PD1⁺CD8⁺ cells after treatment (Post) is 2 to 3 months. Here, the statistical p-value is 0.0201 and the 95% confidence interval of the hazard ratio is 0.034 to 0.760.

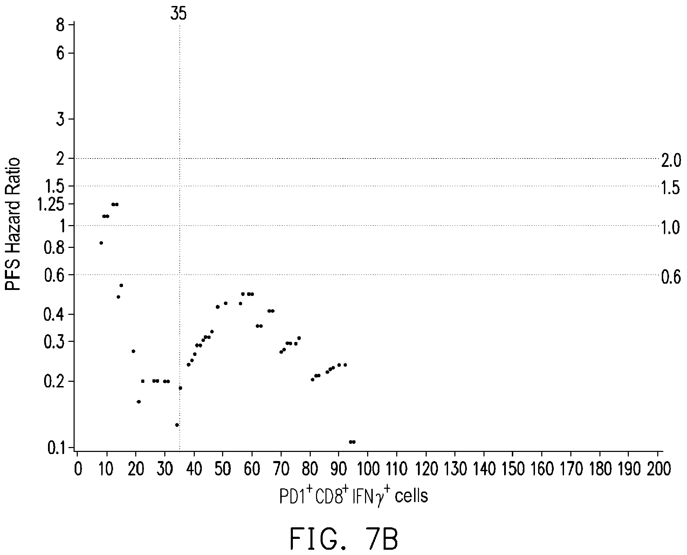

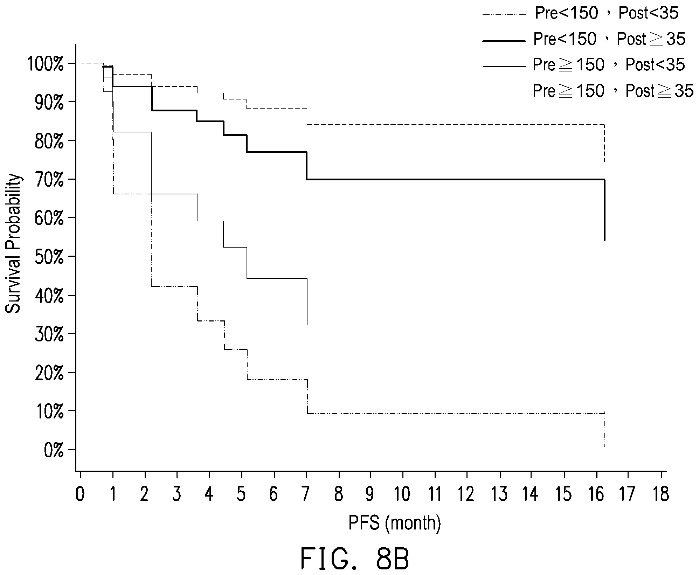

Referring to FIGS. 7B and 8B at the same time, in the analysis results of PD1⁺CD8⁺IFNγ⁺ cells, the first cut-off value is 150 and the second cut-off value is 35. In addition, according to the results of FIG. 8B, when the survival probability is 50%, the progression free survival of the subject having renal cell carcinoma who has 150 or more PD1⁺CD8⁺IFNγ⁺ cells before treatment (Pre) and has 35 or more PD1⁺CD8⁺IFNγ⁺ cells after treatment (Post) is greater than 16 months; the progression free survival of the subject having renal cell carcinoma who has less than 150 PD1⁺CD8⁺IFNγ⁺ cells before treatment (Pre) and has 35 or more PD1⁺CD8⁺IFNγ⁺ cells after treatment (Post) is greater than 16 months; the progression free survival of the subject having renal cell carcinoma who has 150 or more PD1⁺CD8⁺IFNγ⁺ cells before treatment (Pre) and has less than 35 PD1⁺CD8⁺IFNγ⁺ cells after treatment (Post) is 5 to 6 months; the progression free survival of the subject having renal cell carcinoma who has less than 150 PD1⁺CD8⁺IFNγ⁺ cells before treatment (Pre) and has less than 35 PD1⁺CD8⁺IFNγ⁺ cells after treatment (Post) is 2 to 3 months. Here, the statistical p-value is 0.0123 and the 95% confidence interval of the hazard ratio is 0.034 to 0.664.

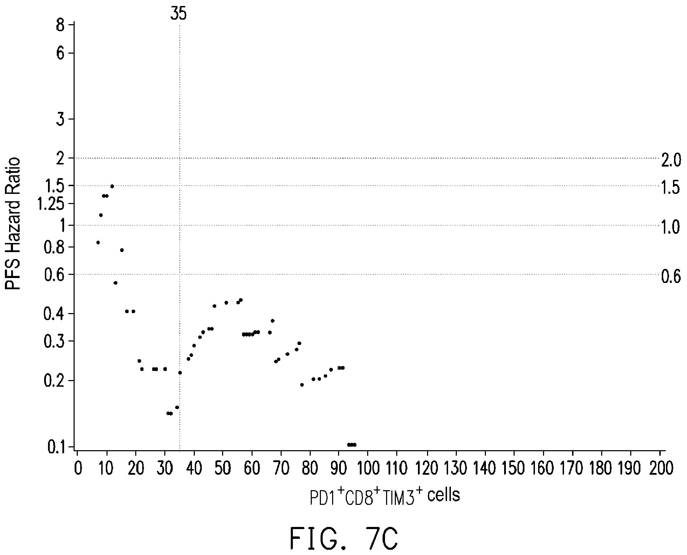

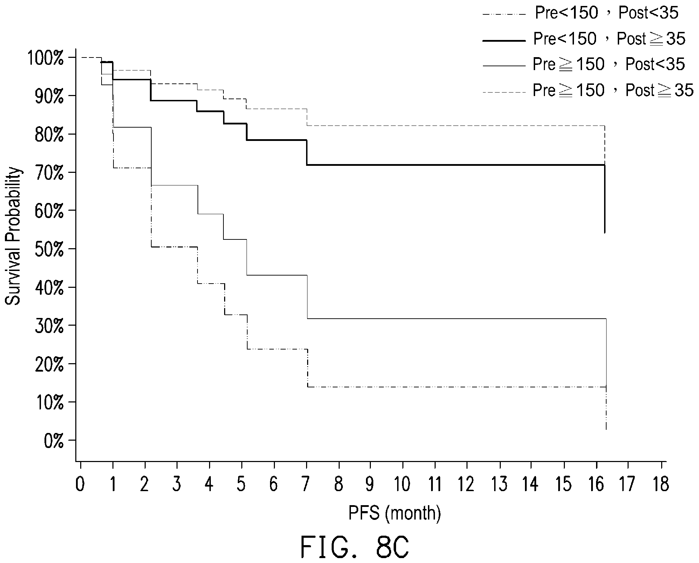

Referring to FIGS. 7C and 8C at the same time, in the analysis results of PD1⁺CD8⁺TIM3⁺ cells, the first cut-off value is 150 and the second cut-off value is 35. In addition, according to the results of FIG. 8C, when the survival probability is 50%, the progression free survival of the subject having renal cell carcinoma who has 150 or more PD1⁺CD8⁺TIM3⁺ cells before treatment (Pre) and has 35 or more PD1⁺CD8⁺TIM3⁺ cells after treatment (Post) is greater than 16 months; the progression free survival of the subject having renal cell carcinoma who has less than 150 PD1⁺CD8⁺TIM3⁺ cells before treatment (Pre) and has 35 or more PD1⁺CD8⁺TIM3⁺ cells after treatment (Post) is greater than 16 months; the progression free survival of the subject having renal cell carcinoma who has 150 or more PD1⁺CD8⁺TIM3⁺ cells before treatment (Pre) and has less than 35 PD1⁺CD8⁺TIM3⁺ cells after treatment (Post) is 5 to 6 months; the progression free survival of the subject having renal cell carcinoma who has less than 150 PD1⁺CD8⁺TIM3⁺ cells before treatment (Pre) and has less than 35 PD1⁺CD8⁺TIM3⁺ cells after treatment (Post) is 3 to 4 months. Here, the statistical p-value is 0.0187 and the 95% confidence interval of the hazard ratio is 0.038 to 0.743.

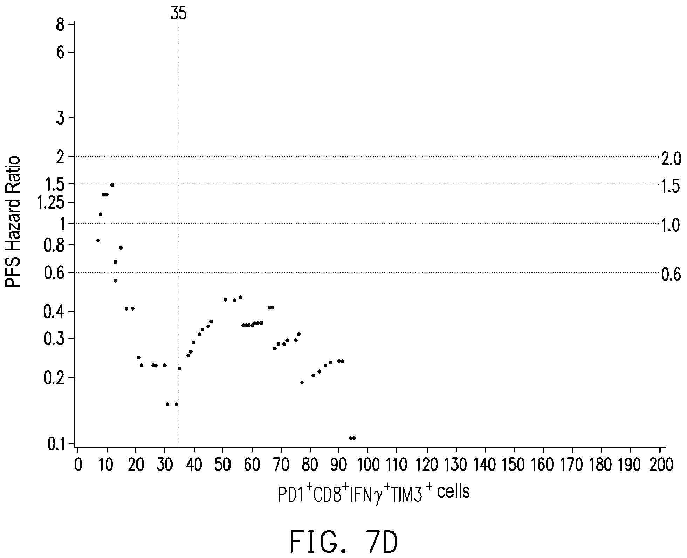

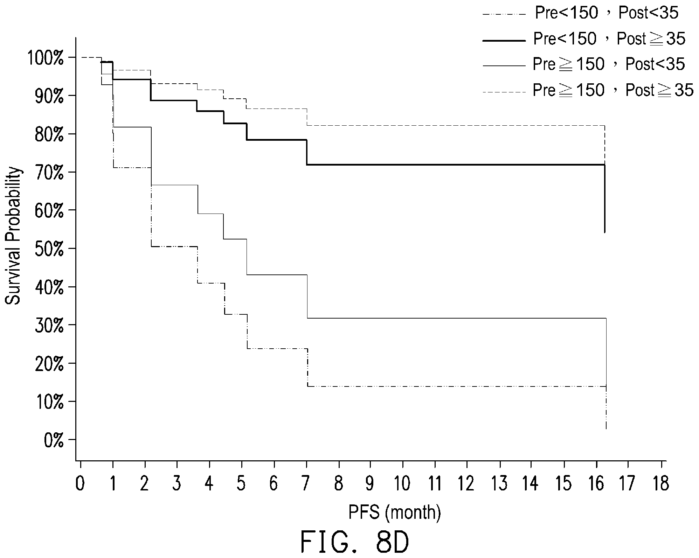

Referring to FIGS. 7D and 8D at the same time, in the analysis results of PD1⁺CD8⁺IFNγ⁺TIM3⁺ cells, the first cut-off value is 150 and the second cut-off value is 35. In addition, according to the results of FIG. 8D, when the survival probability is 50%, the progression free survival of the subject having renal cell carcinoma who has 150 or more PD1⁺CD8⁺IFNγ⁺TIM3⁺ cells before treatment (Pre) and has 35 or more PD1⁺CD8⁺IFNγ⁺TIM3⁺ cells after treatment (Post) is greater than 16 months; the progression free survival of the subject having renal cell carcinoma who has less than 150 PD1⁺CD8⁺IFNγ⁺TIM3⁺ cells before treatment (Pre) and has 35 or more PD1⁺CD8⁺IFNγ⁺TIM3⁺ cells after treatment (Post) is greater than 16 months; the progression free survival of the subject having renal cell carcinoma who has 150 or more PD1⁺CD8⁺IFNγ⁺TIM3⁺ cells before treatment (Pre) and has less than 35 PD1⁺CD8⁺IFNγ⁺TIM3⁺ cells after treatment (Post) is 4 to 5 months; the progression free survival of the subject having renal cell carcinoma who has less than 150 PD1⁺CD8⁺IFNγ⁺TIM3⁺ cells before treatment (Pre) and has less than 35 PD1⁺CD8⁺IFNγ⁺TIM3⁺ cells after treatment (Post) is 2 to 3 months. Here, the statistical p-value is 0.0187 and the 95% confidence interval of the hazard ratio is 0.038 to 0.743.

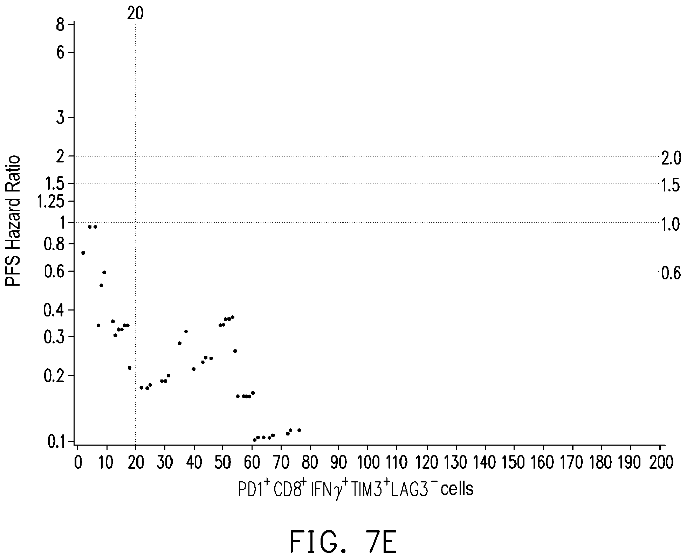

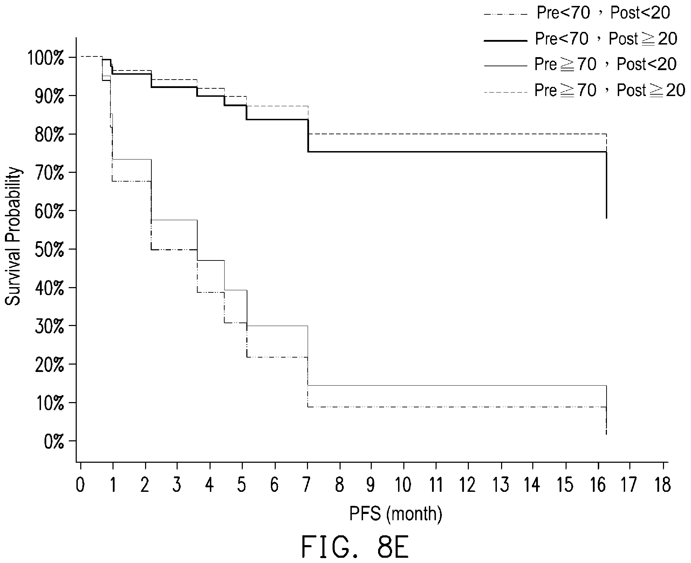

Referring to FIGS. 7E and 8E at the same time, in the analysis results of PD1⁺CD8⁺IFNγ⁺TIM3⁺LAG3⁻ cells, the first cut-off value is 70 and the second cut-off value is 20. In addition, according to the results of FIG. 8E, when the survival probability is 50%, the progression free survival of the subject having renal cell carcinoma who has 70 or more PD1⁺CD8⁺IFNγ⁺TIM3⁺LAG3⁻ cells before treatment (Pre) and has 20 or more PD1⁺CD8⁺IFNγ⁺TIM3⁺LAG3⁻ cells after treatment (Post) is greater than 16 months; the progression free survival of the subject having renal cell carcinoma who has less than 70 PD1⁺CD8⁺IFNγ⁺TIM3⁺LAG3⁻ cells before treatment (Pre) and has 20 or more PD1⁺CD8⁺IFNγ⁺TIM3⁺LAG3⁻ cells after treatment (Post) is greater than 16 months; the progression free survival of the subject having renal cell carcinoma who has 70 or more PD1⁺CD8⁺IFNγ⁺TIM3⁺LAG3⁻ cells before treatment (Pre) and has less than 20 PD1⁺CD8⁺IFNγ⁺TIM3⁺LAG3⁻ cells after treatment (Post) is 3 to 4 months; the progression free survival of the subject having renal cell carcinoma who has less than 70 PD1⁺CD8⁺IFNγ⁺TIM3⁺LAG3⁻ cells before treatment (Pre) and has less than 20 PD1⁺CD8⁺IFNγ⁺TIM3⁺LAG3⁻ cells after treatment (Post) is 2 to 3 months. Here, the statistical p-value is 0.0381 and the 95% confidence interval of the hazard ratio is 1.093 to 23.386.

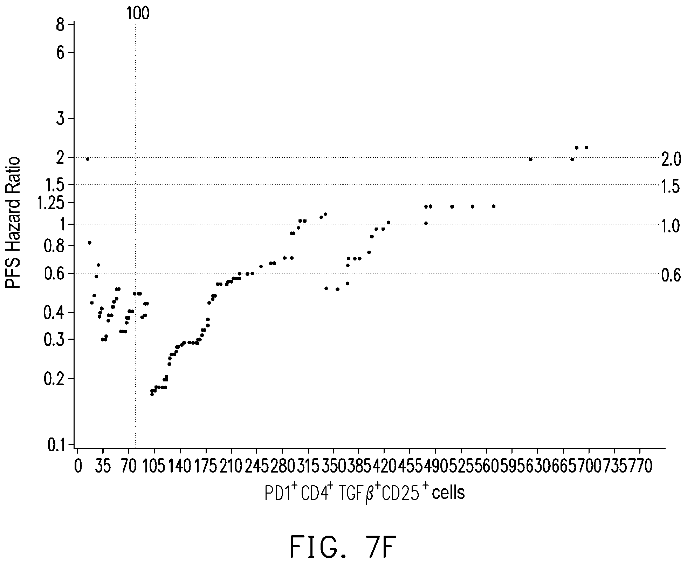

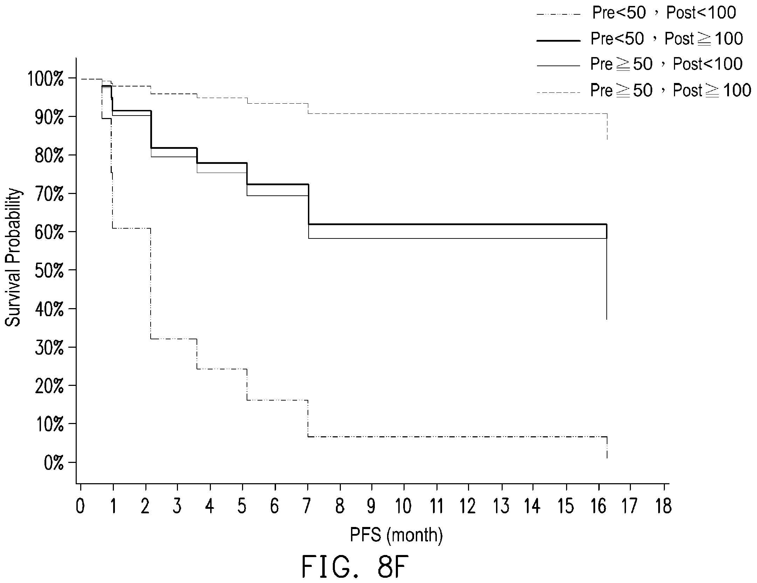

Referring to FIGS. 7F and 8F at the same time, in the analysis results of PD1⁺CD4⁺TGFβ⁺CD25⁺ cells, the first cut-off value is 50 and the second cut-off value is 100. In addition, according to the results of FIG. 8F, when the survival probability is 50%, the progression free survival of the subject having renal cell carcinoma who has 50 or more PD1⁺CD4⁺TGFβ⁺CD25⁺ cells before treatment (Pre) and has 100 or more PD1⁺CD4⁺TGFβ⁺CD25⁺ cells after treatment (Post) is greater than 16 months; the progression free survival of the subject having renal cell carcinoma who has less than 50 PD1⁺CD4⁺TGFβ⁺CD25⁺ cells before treatment (Pre) and has 100 or more PD1⁺CD4⁺TGFβ⁺CD25⁺ cells after treatment (Post) is greater than 16 months; the progression free survival of the subject having renal cell carcinoma who has 50 or more PD1⁺CD4⁺TGFβ⁺CD25⁺ cells before treatment (Pre) and has less than 100 PD1⁺CD4⁺TGFβ⁺CD25⁺ cells after treatment (Post) is greater than 16 months; the progression free survival of the subject having renal cell carcinoma who has less than 50 PD1⁺CD4⁺TGFβ⁺CD25⁺ cells before treatment (Pre) and has less than 100 PD1⁺CD4⁺TGFβ⁺CD25⁺ cells after treatment (Post) is 2 to 3 months. Here, the statistical p-value is 0.0514 and the 95% confidence interval of the hazard ratio is 0.030 to 1.011.

Based on the above, according to the results of FIGS. 7A to 7F and FIGS. 8A to 8F, for the subjects having renal cell carcinoma who have received the anti-PD-1 / PD-L1 immunotherapy, when the number of immune cells of the subjects having renal cell carcinoma is greater than or equal to the first cut-off value before treatment (Pre) and is greater than or equal to the second cut-off value after treatment (Post), or when the number of immune cells of the subjects having renal cell carcinoma is less than the first cut-off value before treatment (Pre) and is greater than or equal to the second cut-off value after treatment (Post), the subjects having renal cell carcinoma have good prognosis and the anti-PD-1 / PD-L1 immunotherapy shows better treatment effectiveness on the subjects having renal cell carcinoma. However, for the subjects having renal cell carcinoma who have received the anti-PD-1 / PD-L1 immunotherapy, when the number of immune cells of the subjects having renal cell carcinoma is greater than or equal to the first cut-off value before treatment (Pre) and is less than the second cut-off value after treatment (Post), or when the number of immune cells of the subjects having renal cell carcinoma is less than the first cut-off value before treatment (Pre) and is less than the second cut-off value after treatment (Post), the subjects having renal cell carcinoma have worse prognosis and the anti-PD-1 / PD-L1 immunotherapy shows worse treatment effectiveness on the subjects having hepatocellular carcinoma. Here, the above immune cells include PD1⁺CD8⁺ cells, PD1⁺CD8⁺IFNγ⁺ cells, PD1⁺CD8⁺TIM3⁺ cells, PD1⁺CD8⁺IFNγ⁺TIM3⁺ cells, PD1⁺CD8⁺IFNγ⁺TIM3⁺LAG3⁻ cells, and PD1⁺CD4⁺TGFβ⁺CD25⁺ cells, but is not limited thereto.

### Experimental 5: predicting anti-PD-1 / PD-L1 immunotherapy response of subjects having urothelial cancer after receiving the anti-PD-1 / PD-Llimmunotherapy

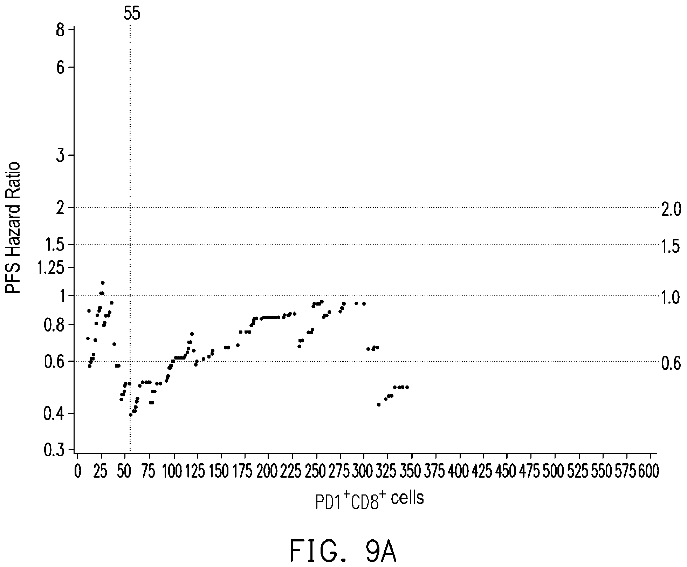

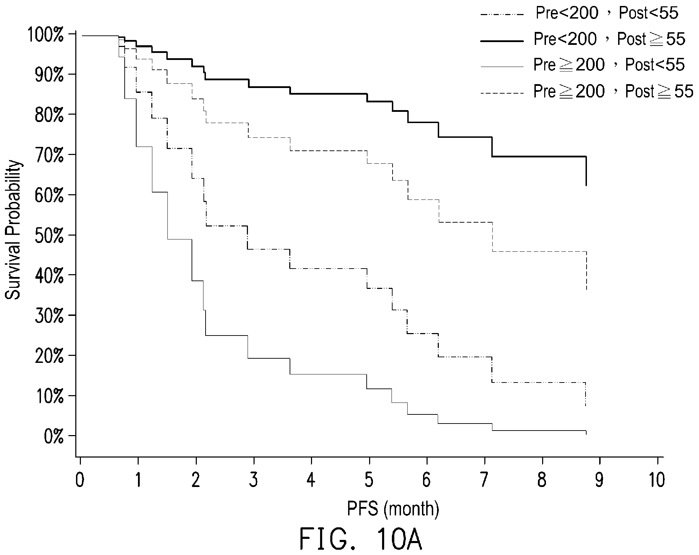

FIG. 9A to FIG. 9E are scatter plots of the number of immune cells of the subject having urothelial cancer after receiving the anti-PD1 / PD-L1 immunotherapy between the end of one round of treatment until the start of the next round of treatment and the hazard ratio of the progression free survival. FIG. 10A to FIG. 10E are Cox regression models of the progression free survival and the survival probability of the subject having urothelial cancer after receiving the anti-PD1 / PD-L1 immunotherapy.

Referring to FIGS. 9A and 10A at the same time, in the analysis results of PD1⁺CD8⁺ cells, the first cut-off value is 200 and the second cut-off value is 55. In addition, according to the results of FIG. 10A, when the survival probability is 50%, the progression free survival of the subject having urothelial cancer who has less than 200 PD1⁺CD8⁺ cells before treatment (Pre) and has 55 or more PD1⁺CD8⁺ cells after treatment (Post) is greater than 8 months; the progression free survival of the subject having urothelial cancer who has 200 or more PD1⁺CD8⁺ cells before treatment (Pre) and has 55 or more PD1⁺CD8⁺ cells after treatment (Post) is 7 to 8 months; the progression free survival of the subject having urothelial cancer who has less than 200 PD1⁺CD8⁺ cells before treatment (Pre) and has less than 55 PD1⁺CD8⁺ cells after treatment (Post) is 2 to 3 months; the progression free survival of the subject having urothelial cancer who has 200 or more PD1⁺CD8⁺ cells before treatment (Pre) and has less than 55 PD1⁺CD8⁺ cells after treatment (Post) is 1 to 2 months. Here, the statistical p-value is 0.0141 and the 95% confidence interval of the hazard ratio is 0.046 to 0.707.

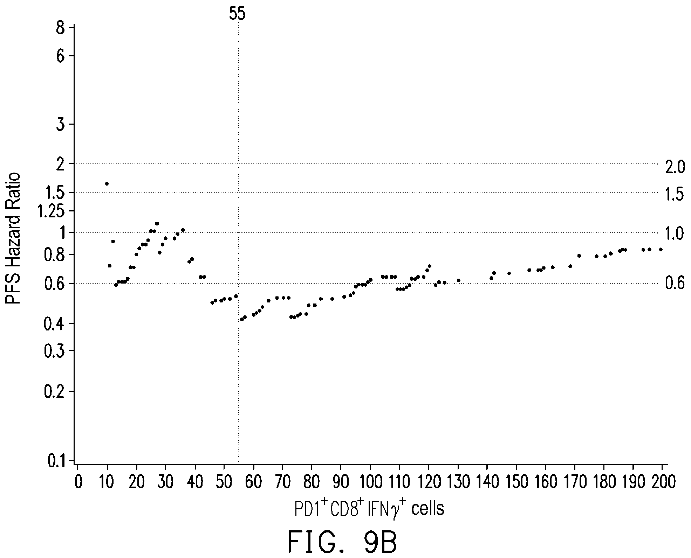

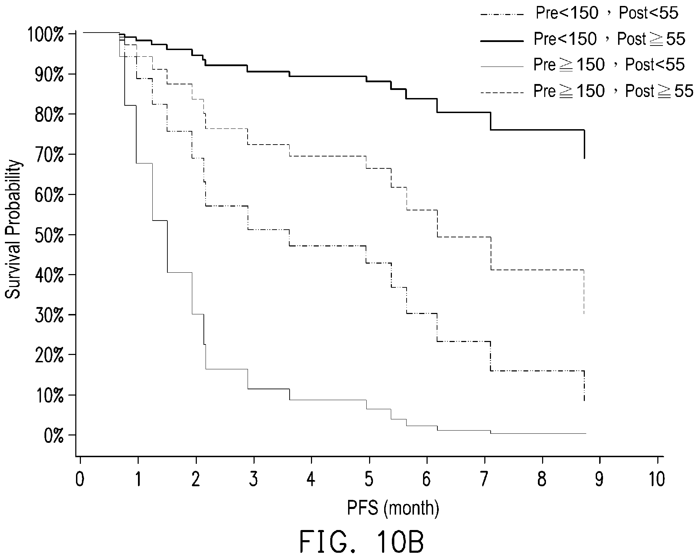

Referring to FIGS. 9B and 10B at the same time, in the analysis results of PD1⁺CD8⁺IFNγ⁺ cells, the first cut-off value is 150 and the second cut-off value is 55. In addition, according to the results of FIG. 10B, when the survival probability is 50%, the progression free survival of the subject having urothelial cancer who has less than 150 PD1⁺CD8⁺IFNγ⁺ cells before treatment (Pre) and has 55 or more PD1⁺CD8⁺IFNγ⁺ cells after treatment (Post) is greater than 8 months; the progression free survival of the subject having urothelial cancer who has 150 or more PD1⁺CD8⁺IFNγ⁺ cells before treatment (Pre) and has 55 or more PD1⁺CD8⁺IFNγ⁺ cells after treatment (Post) is 6 to 7 months; the progression free survival of the subject having urothelial cancer who has less than 150 PD1⁺CD8⁺IFNγ⁺ cells before treatment (Pre) and has less than 55 PD1⁺CD8⁺IFNγ⁺ cells after treatment (Post) is 3 to 4 months; the progression free survival of the subject having urothelial cancer who has 150 or more PD1⁺CD8⁺IFNγ⁺ cells before treatment (Pre) and has less than 55 PD1⁺CD8⁺IFNγ⁺ cells after treatment (Post) is 1 to 2 months. Here, the statistical p-value is 0.0063 and the 95% confidence interval of the hazard ratio is 0.039 to 0.585.

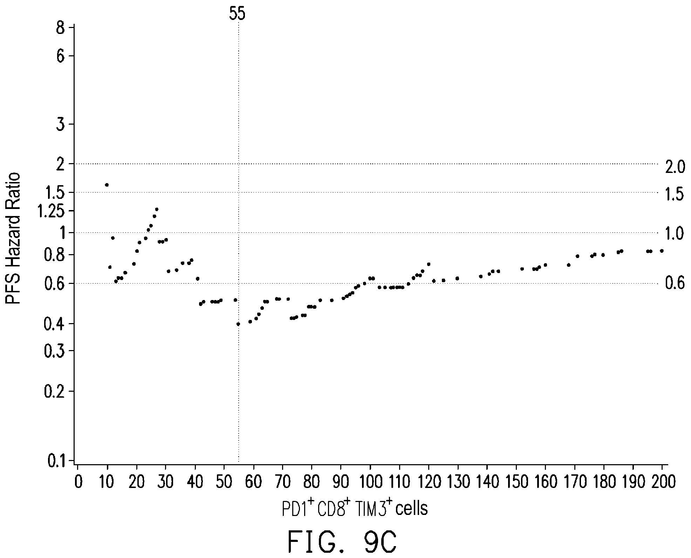

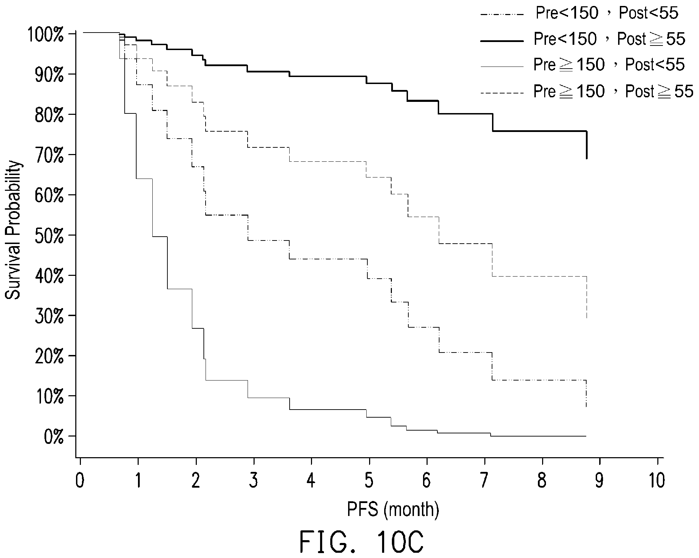

Referring to FIGS. 9C and 10C at the same time, in the analysis results of PD1⁺CD8⁺TIM3⁺ cells, the first cut-off value is 150 and the second cut-off value is 55. In addition, according to the results of FIG. 10C, when the survival probability is 50%, the progression free survival of the subject having urothelial cancer who has less than 150 PD1⁺CD8⁺TIM3⁺ cells before treatment (Pre) and has 55 or more PD1⁺CD8⁺TIM3⁺ cells after treatment (Post) is greater than 8 months; the progression free survival of the subject having urothelial cancer who has 150 or more PD1⁺CD8⁺TIM3⁺ cells before treatment (Pre) and has 55 or more PD1⁺CD8⁺TIM3⁺ cells after treatment (Post) is 6 to 7 months; the progression free survival of the subject having urothelial cancer who has less than 150 PD1⁺CD8⁺TIM3⁺ cells before treatment (Pre) and has less than 55 PD1⁺CD8⁺TIM3⁺ cells after treatment (Post) is 2 to 3 months; the progression free survival of the subject having urothelial cancer who has 150 or more PD1⁺CD8⁺TIM3⁺ cells before treatment (Pre) and has less than 55 PD1⁺CD8⁺TIM3⁺ cells after treatment (Post) is 1 to 2 months. Here, the statistical p-value is 0.0085 and the 95% confidence interval of the hazard ratio is 0.033 to 0.608.

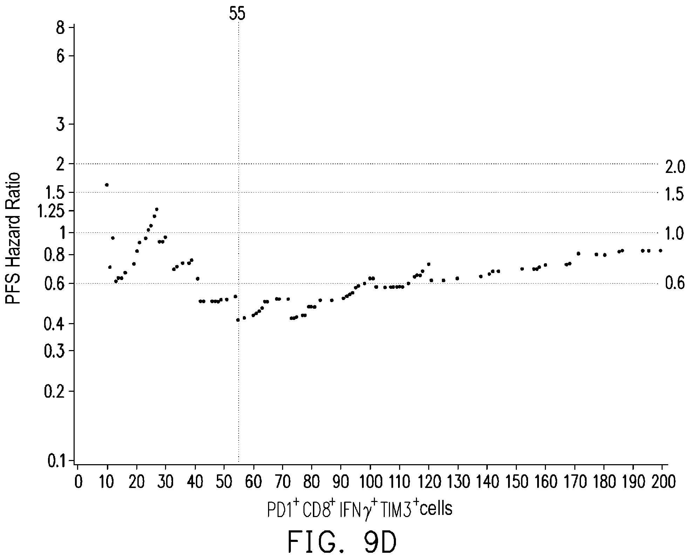

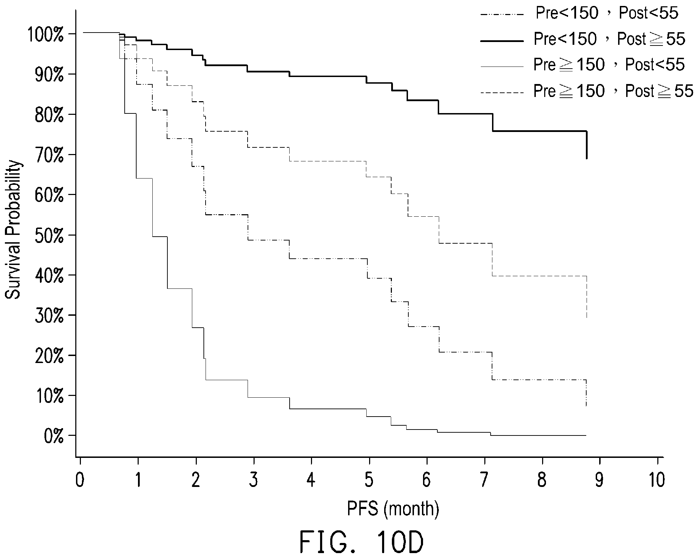

Referring to FIGS. 9D and 10D at the same time, in the analysis results of PD1⁺CD8⁺IFNγ⁺TIM3⁺ cells, the first cut-off value is 150 and the second cut-off value is 55. In addition, according to the results of FIG. 10D, when the survival probability is 50%, the progression free survival of the subject having urothelial cancer who has less than 150 PD1⁺CD8⁺IFNγ⁺TIM3⁺ cells before treatment (Pre) and has 55 or more PD1⁺CD8⁺IFNγ⁺TIM3⁺ cells after treatment (Post) is greater than 8 months; the progression free survival of the subject having urothelial cancer who has 150 or more PD 1⁺CD8⁺IFNγ⁺TIM3⁺ cells before treatment (Pre) and has 55 or more PD1⁺CD8⁺IFNγ⁺TIM3⁺ cells after treatment (Post) is 6 to 7 months; the progression free survival of the subject having urothelial cancer who has less than 150 PD1⁺CD8⁺IFNγ⁺TIM3⁺ cells before treatment (Pre) and has less than 55 PD1⁺CD8⁺IFNγ⁺TIM3⁺ cells after treatment (Post) is 2 to 3 months; the progression free survival of the subject having urothelial cancer who has 150 or more PD1⁺CD8⁺IFNγ⁺TIM3⁺ cells before treatment (Pre) and has less than 55 PD1⁺CD8⁺IFNγ⁺TIM3⁺ cells after treatment (Post) is 1 to 2 months. Here, the statistical p-value is 0.0085 and the 95% confidence interval of the hazard ratio is 0.033 to 0.608.

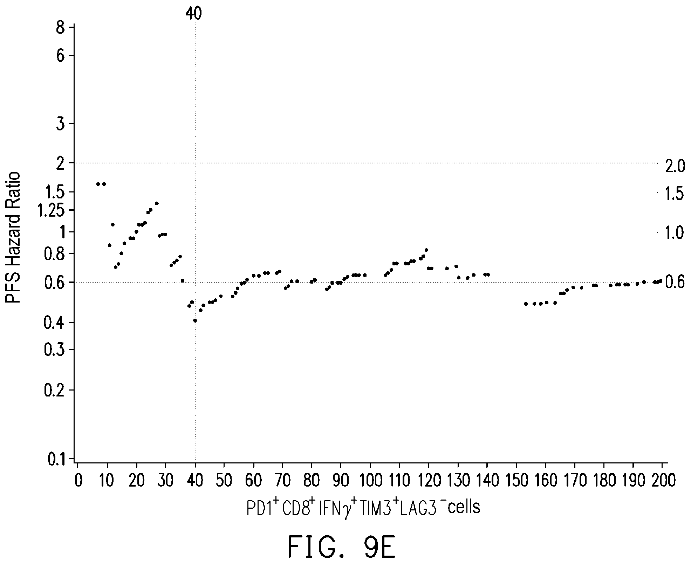

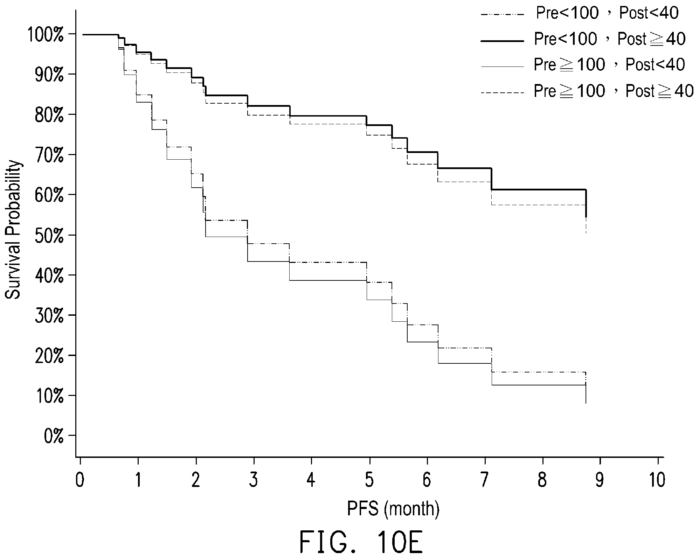

Referring to FIGS. 9E and 10E at the same time, in the analysis results of PD1⁺CD8⁺IFNγ⁺TIM3⁺LAG3⁻ cells, the first cut-off value is 100 and the second cut-off value is 40. In addition, according to the results of FIG. 10E, when the survival probability is 50%, the progression free survival of the subject having urothelial cancer who has less than 100 PD1⁺CD8⁺IFNγ⁺TIM3⁺LAG3⁻ cells before treatment (Pre) and has 40 or more PD1⁺CD8⁺IFNγ⁺TIM3⁺LAG3⁻ cells after treatment (Post) is greater than 8 months; the progression free survival of the subject having urothelial cancer who has 100 or more PD1⁺CD8⁺IFNγ⁺TIM3⁺LAG3⁻ cells before treatment (Pre) and has 40 or more PD1⁺CD8⁺IFNγ⁺TIM3⁺LAG3⁻ cells after treatment (Post) is greater than 8 months; the progression free survival of the subject having urothelial cancer who has less than 100 PD1⁺CD8⁺IFNγ⁺TIM3⁺LAG3⁻ cells before treatment (Pre) and has less than 40 PD1⁺CD8⁺IFNγ⁺TIM3⁺LAG3⁻ cells after treatment (Post) is 2 to 3 months; the progression free survival of the subject having urothelial cancer who has 100 or more PD1⁺CD8⁺IFNγ⁺TIM3⁺LAG3⁻ cells before treatment (Pre) and has less than 40 PD1⁺CD8⁺ cells after treatment (Post) is 2 to 3 months. Here, the statistical p-value is 0.0274 and the 95% confidence interval of the hazard ratio is 0.082 to 0.863.

Based on the above, according to the results of FIGS. 9A to 9E and FIGS. 10A to 10E, for the subjects having urothelial cancer who have received the anti-PD-1 / PD-L1 immunotherapy, when the number of immune cells of the subjects having urothelial cancer is less than the first cut-off value before treatment (Pre) and is greater than or equal to the second cut-off value after treatment (Post), or when the number of immune cells of the subjects having urothelial cancer is greater than or equal to the first cut-off value before treatment (Pre) and is greater than or equal to the second cut-off value after treatment (Post), the subjects having urothelial cancer have good prognosis and the anti-PD-1 / PD-L1 immunotherapy shows better treatment effectiveness on the subjects having urothelial cancer. However, for the subjects having urothelial cancer who have received the anti-PD-1 / PD-L1 immunotherapy, when the number of immune cells of the subjects having urothelial cancer is less than the first cut-off value before treatment (Pre) and is less than the second cut-off value after treatment (Post), or when the number of immune cells of the subjects having urothelial cancer is greater than or equal to the first cut-off value before treatment (Pre) and is less than the second cut-off value after treatment (Post), the subjects having urothelial cancer have worse prognosis and the anti-PD-1 / PD-L1 immunotherapy shows worse treatment effectiveness on the subjects having hepatocellular carcinoma. Here, the above immune cells include PD1⁺CD8⁺ cells, PD1⁺CD8⁺IFNγ⁺ cells, PD1⁺CD8⁺TIM3⁺ cells, PD1⁺CD8⁺IFNγ⁺TIM3⁺ cells, and PD1⁺CD8⁺IFNγ⁺TIM3⁺LAG3⁻ cells, but is not limited thereto.

In summary of the above, in the method for predicting the anti-PD-1/PD-L1 immunotherapy response of the subject having cancer of the embodiment of the invention, by detecting the number of immune cells in the peripheral blood samples of the subject having cancer and comparing the number of immune cells with the first cut-off value/second cut-off value, it may predict whether the subject having cancer benefits from the anti-PD-1/PD-L1 immunotherapy before the first treatment, and may obtain the treatment effectiveness of the anti-PD-1/PD-Llimmunotherapy on the subject having cancer soon after the treatment and before the next treatment. Furthermore, compared with conventional technology which is necessary to take tumor cells of subjects having cancer for analysis and thus make the subjects having cancer suffer greater pains, the method provided from the embodiment of the present invention only needs to collect specific immune cells by blood drawing and calculate the number of the immune cells, which may greatly shorten test time and is more convenient for the subjects having cancer. Therefore, the method provided from the embodiments of the present invention has the advantages of saving cost and time for the subjects receiving immunotherapy.

## Claims

1. A method for predicting immunotherapy response of a subject having cancer, comprising:
obtaining a peripheral blood sample from the subject having cancer before receiving the immunotherapy;
detecting the number of immune cells in the peripheral blood sample of the subject having cancer; and
comparing the number of immune cells with a first cut-off value to indicate whether the subject having cancer benefits from the immunotherapy,
wherein the first cut-off value is determined by the following steps: performing a statistical analysis of a correlation between the number of immune cells in a group of subjects having cancer and an expected risk of disease progression in the group of subjects having cancer, and then obtaining a statistically significant value used to define the correlation.

2. The method of claim 1, wherein the immunotherapy comprises anti-PD-1/PD-L1 immunotherapy.

3. The method of claim 1, wherein the immune cells express at least one marker of: PD1, CD8, CD4, IFN-γ, TIM3, LAG3, CD25, TGF-β.

4. The method of claim 1, wherein the cancer is hepatocellular carcinoma and the immune cells are selected from the group consisting of PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CDS⁺IFNγ⁺LAG3⁺ cells and PD1⁺CD8⁺IFNγ⁺TIM3⁺LAG3⁻ cells.

5. The method of claim 1, wherein the cancer is renal cell carcinoma and the immune cells are selected from the group consisting of PD1⁺CD4⁺TGFβ⁺CD25⁺ cells, PD1⁺CD4⁺TGFβ⁺CD25⁺LAG3⁻ cells and PD1⁺CD4⁺TGFβ⁺CD25⁺LAG3⁺ cells.

6. The method of claim 1, wherein the first cut-off value divides the subject into group A and group B according to a pattern of hazard ratio, wherein the subject classified into group A has a good prognosis, and the subject classified into group B has worse prognosis.

7. The method of claim 6, wherein the hazard ratio is measured by a Cox regression model of a survival time in the group of subjects having cancer versus a survival probability in the group of subjects having cancer.

8. A method for predicting immunotherapy response of a subject having cancer, comprising:
obtaining a peripheral blood sample from the subject receiving the immunotherapy between the end of one round of treatment until the start of the next round of treatment;
detecting the number of immune cells in the peripheral blood sample of the subject having cancer; and
comparing the number of immune cells with a second cut-off value to obtain the treatment effectiveness of the immunotherapy on the subject having cancer,
wherein the second cut-off value is determined by the following steps: performing a statistical analysis of a correlation between the number of immune cells in a group of subjects having cancer and an expected risk of disease progression in the group of subjects having cancer, and then obtaining a statistically significant value used to define the correlation.

9. The method of claim 8, wherein the immunotherapy comprises anti-PD-1/PD-L1 immunotherapy.

10. The method of claim 8, wherein the immune cells express at least one marker of: PD1, CD8, CD4, IFN-γ, TIM3, LAG3, CD25, TGF-β.

11. The method of claim 8, wherein the cancer is hepatocellular carcinoma, and the immune cells are selected from the group consisting of PD1⁺CD8⁺ cells, PD1⁺CD8⁺IFNγ⁺ cells, PD1⁺CD8⁺TIM3⁺ cells, PD1⁺CD8⁺LAG3⁻ cells, PD1⁺CD8⁺LAG3⁺ cells, PD1⁺CD8⁺IFNγ⁺LAG3⁺ cells, PD1⁺CD8⁺IFNγ⁺LAG3⁻ cells, PD1⁺CD8⁺IFNγ⁺TIM3⁺LAG3⁺ cells and PD1⁺CD8⁺IFNγ⁺TIM3⁺LAG3⁻ cells.

12. The method of claim 8, wherein the cancer is renal cell carcinoma, and the immune cells are selected from the group consisting of PD1⁺CD8⁺ cells, PD1⁺CD8⁺TIM3⁺ cells, PD1⁺CD8⁺IFNγ⁺ cells PD1⁺CD8⁺IFNγ⁺TIM3⁺ cells, PD1⁺CD8⁺IFNγ⁺TIM3⁺LAG3⁻ cells and PD1⁺CD4⁺TGFβ⁺CD25⁺ cells.

13. The method of claim 8, wherein the cancer is urothelial cancer, and the immune cells are selected from the group consisting of PD1⁺CD8⁺ cells, PD1⁺CD8⁺TIM3⁺ cells, PD1⁺CD8⁺IFNγ⁺ cells, PD1⁺CD8⁺IFNγ⁺TIM3⁺ cells and PD1⁺CD8⁺IFNγ⁺TIM3⁺LAG3⁻ cells.

14. The method of claim 8, wherein the second cut-off value divided the subject into group A and group B according to a pattern of hazard ratio, wherein the subject classified into group A has a good prognosis, and the subject classified into group B has worse prognosis.

15. The method of claim 14, wherein the hazard ratio is measured by a Cox regression model of a survival time in the group of subjects having cancer versus a survival probability in the group of subjects having cancer.
